# EUROPEAN PATENT APPLICATION

(11) **EP 3 738 978 A1**
(43) Date of publication of application: **18.11.2020**
(21) Application number: 19738515.6
(22) Date of filing: 11.01.2019
(51) Int. Cl.: C07K 14/755, C12N 9/64, C07K 19/00, A61K 47/54, A61K 47/60

(54) **IN-VIVO RELEASE SUSTAINED RECOMBINANT COAGULATION FACTOR VIII AND PREPARATION METHOD THEREFOR**

(30) Priority: 12.01.2018 KR 20180004589
(71) Applicant: Mogam Institute for Biomedical Research, Yongin-si, Gyeonggi-do 16924 (KR)
(72) Inventor: KANG, Kwan Yub, Yongin-si Gyeonggi-do 16924 (KR); KIM, Taeyoon, Yongin-si Gyeonggi-do 16924 (KR); RYU, Jae Hwan, Yongin-si Gyeonggi-do 16924 (KR); KIM, Yeon Jung, Yongin-si Gyeonggi-do 16924 (KR); KIM, Yong Jae, Yongin-si Gyeonggi-do 16924 (KR)
(74) Representative: HGF
(86) International application number: PCT/KR2019/000479
(87) International publication number: WO 2019/139416

(57) **Abstract**

The present invention provides an *in-vivo* release-sustained recombinant coagulation factor VIII in which a biocompatible polymer is conjugated to at least one sugar chain terminus, and a preparation method therefor. An *in-vivo* release-sustained recombinant coagulation factor VIII according to the present invention has a protein surface modified through a method more elaborate and safer than conventional glycoconjugation techniques and enjoys the advantage of decreasing in immunogenicity and increasing in *in-vivo* sustainability.

<110>    Mogam Institute for Biomedical Research

<120>    LONG-ACTING RECOMBINANT BLOOD COAGULATION FACTOR VIII AND
         MENUFACTURING METHOD THEREOF

<130>    812139

<150>    KR 2018-0004589
<151>    2018-01-12

<160>    15

<170>    KoPatentIn 3.0

<210>    1
<211>    2332
<212>    PRT
<213>    Homo sapiens

<400>    1
Ala Thr Arg Arg Tyr Tyr Leu Gly Ala Val Glu Leu Ser Trp Asp Tyr
  1               5                  10                  15

Met Gln Ser Asp Leu Gly Glu Leu Pro Val Asp Ala Arg Phe Pro Pro
             20                  25                  30

Arg Val Pro Lys Ser Phe Pro Phe Asn Thr Ser Val Val Tyr Lys Lys
         35                  40                  45

Thr Leu Phe Val Glu Phe Thr Asp His Leu Phe Asn Ile Ala Lys Pro
     50                  55                  60

Arg Pro Pro Trp Met Gly Leu Leu Gly Pro Thr Ile Gln Ala Glu Val
 65                  70                  75                  80

Tyr Asp Thr Val Val Ile Thr Leu Lys Asn Met Ala Ser His Pro Val
                 85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg

225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe

                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His

            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Val Ser Ile Ser Leu Leu Lys Thr Asn Lys Thr Ser Asn Asn Ser Ala
        995                 1000                1005

Thr Asn Arg Lys Thr His Ile Asp Gly Pro Ser Leu Leu Ile Glu Asn
    1010                1015                1020

Ser Pro Ser Val Trp Gln Asn Ile Leu Glu Ser Asp Thr Glu Phe Lys
1025                1030                1035                1040

Lys Val Thr Pro Leu Ile His Asp Arg Met Leu Met Asp Lys Asn Ala
                1045                1050                1055

Thr Ala Leu Arg Leu Asn His Met Ser Asn Lys Thr Thr Ser Ser Lys
            1060                1065                1070

Asn Met Glu Met Val Gln Gln Lys Lys Glu Gly Pro Ile Pro Pro Asp
        1075                1080                1085

Ala Gln Asn Pro Asp Met Ser Phe Phe Lys Met Leu Phe Leu Pro Glu
    1090                1095                1100

Ser Ala Arg Trp Ile Gln Arg Thr His Gly Lys Asn Ser Leu Asn Ser
1105                1110                1115                1120

Gly Gln Gly Pro Ser Pro Lys Gln Leu Val Ser Leu Gly Pro Glu Lys
                1125                1130                1135

Ser Val Glu Gly Gln Asn Phe Leu Ser Glu Lys Asn Lys Val Val Val
            1140                1145                1150

Gly Lys Gly Glu Phe Thr Lys Asp Val Gly Leu Lys Glu Met Val Phe
        1155                1160                1165

Pro Ser Ser Arg Asn Leu Phe Leu Thr Asn Leu Asp Asn Leu His Glu
    1170                1175                1180

Asn Asn Thr His Asn Gln Glu Lys Lys Ile Gln Glu Glu Ile Glu Lys
1185                1190                1195                1200

Lys Glu Thr Leu Ile Gln Glu Asn Val Val Leu Pro Gln Ile His Thr
                1205                1210                1215

Val Thr Gly Thr Lys Asn Phe Met Lys Asn Leu Phe Leu Leu Ser Thr
            1220                1225                1230

Arg Gln Asn Val Glu Gly Ser Tyr Asp Gly Ala Tyr Ala Pro Val Leu

1235                1240                1245

Gln Asp Phe Arg Ser Leu Asn Asp Ser Thr Asn Arg Thr Lys Lys His
    1250                1255                1260

Thr Ala His Phe Ser Lys Lys Gly Glu Glu Glu Asn Leu Glu Gly Leu
1265                1270                1275                1280

Gly Asn Gln Thr Lys Gln Ile Val Glu Lys Tyr Ala Cys Thr Thr Arg
                1285                1290                1295

Ile Ser Pro Asn Thr Ser Gln Gln Asn Phe Val Thr Gln Arg Ser Lys
            1300                1305                1310

Arg Ala Leu Lys Gln Phe Arg Leu Pro Leu Glu Glu Thr Glu Leu Glu
        1315                1320                1325

Lys Arg Ile Ile Val Asp Asp Thr Ser Thr Gln Trp Ser Lys Asn Met
    1330                1335                1340

Lys His Leu Thr Pro Ser Thr Leu Thr Gln Ile Asp Tyr Asn Glu Lys
1345                1350                1355                1360

Glu Lys Gly Ala Ile Thr Gln Ser Pro Leu Ser Asp Cys Leu Thr Arg
                1365                1370                1375

Ser His Ser Ile Pro Gln Ala Asn Arg Ser Pro Leu Pro Ile Ala Lys
            1380                1385                1390

Val Ser Ser Phe Pro Ser Ile Arg Pro Ile Tyr Leu Thr Arg Val Leu
        1395                1400                1405

Phe Gln Asp Asn Ser Ser His Leu Pro Ala Ala Ser Tyr Arg Lys Lys
    1410                1415                1420

Asp Ser Gly Val Gln Glu Ser Ser His Phe Leu Gln Gly Ala Lys Lys
1425                1430                1435                1440

Asn Asn Leu Ser Leu Ala Ile Leu Thr Leu Glu Met Thr Gly Asp Gln
                1445                1450                1455

Arg Glu Val Gly Ser Leu Gly Thr Ser Ala Thr Asn Ser Val Thr Tyr
            1460                1465                1470

Lys Lys Val Glu Asn Thr Val Leu Pro Lys Pro Asp Leu Pro Lys Thr
        1475                1480                1485

Ser Gly Lys Val Glu Leu Leu Pro Lys Val His Ile Tyr Gln Lys Asp
    1490                1495                1500

Leu Phe Pro Thr Glu Thr Ser Asn Gly Ser Pro Gly His Leu Asp Leu
1505                1510                1515                1520

Val Glu Gly Ser Leu Leu Gln Gly Thr Glu Gly Ala Ile Lys Trp Asn
                1525                1530                1535

Glu Ala Asn Arg Pro Gly Lys Val Pro Phe Leu Arg Val Ala Thr Glu
            1540                1545                1550

Ser Ser Ala Lys Thr Pro Ser Lys Leu Leu Asp Pro Leu Ala Trp Asp
        1555                1560                1565

Asn His Tyr Gly Thr Gln Ile Pro Lys Glu Glu Trp Lys Ser Gln Glu

1570                1575                1580

Lys Ser Pro Glu Lys Thr Ala Phe Lys Lys Lys Asp Thr Ile Leu Ser
1585                1590                1595                1600

Leu Asn Ala Cys Glu Ser Asn His Ala Ile Ala Ala Ile Asn Glu Gly
                1605                1610                1615

Gln Asn Lys Pro Glu Ile Glu Val Thr Trp Ala Lys Gln Gly Arg Thr
            1620                1625                1630

Glu Arg Leu Cys Ser Gln Asn Pro Pro Val Leu Lys Arg His Gln Arg
        1635                1640                1645

Glu Ile Thr Arg Thr Thr Leu Gln Ser Asp Gln Glu Glu Ile Asp Tyr
    1650                1655                1660

Asp Asp Thr Ile Ser Val Glu Met Lys Lys Glu Asp Phe Asp Ile Tyr
1665                1670                1675                1680

Asp Glu Asp Glu Asn Gln Ser Pro Arg Ser Phe Gln Lys Lys Thr Arg
                1685                1690                1695

His Tyr Phe Ile Ala Ala Val Glu Arg Leu Trp Asp Tyr Gly Met Ser
            1700                1705                1710

Ser Ser Pro His Val Leu Arg Asn Arg Ala Gln Ser Gly Ser Val Pro
        1715                1720                1725

Gln Phe Lys Lys Val Val Phe Gln Glu Phe Thr Asp Gly Ser Phe Thr
    1730                1735                1740

Gln Pro Leu Tyr Arg Gly Glu Leu Asn Glu His Leu Gly Leu Leu Gly
1745                1750                1755                1760

Pro Tyr Ile Arg Ala Glu Val Glu Asp Asn Ile Met Val Thr Phe Arg
                1765                1770                1775

Asn Gln Ala Ser Arg Pro Tyr Ser Phe Tyr Ser Ser Leu Ile Ser Tyr
            1780                1785                1790

Glu Glu Asp Gln Arg Gln Gly Ala Glu Pro Arg Lys Asn Phe Val Lys
        1795                1800                1805

Pro Asn Glu Thr Lys Thr Tyr Phe Trp Lys Val Gln His His Met Ala
    1810                1815                1820

Pro Thr Lys Asp Glu Phe Asp Cys Lys Ala Trp Ala Tyr Phe Ser Asp
1825                1830                1835                1840

Val Asp Leu Glu Lys Asp Val His Ser Gly Leu Ile Gly Pro Leu Leu
                1845                1850                1855

Val Cys His Thr Asn Thr Leu Asn Pro Ala His Gly Arg Gln Val Thr
            1860                1865                1870

Val Gln Glu Phe Ala Leu Phe Phe Thr Ile Phe Asp Glu Thr Lys Ser
        1875                1880                1885

Trp Tyr Phe Thr Glu Asn Met Glu Arg Asn Cys Arg Ala Pro Cys Asn
    1890                1895                1900

Ile Gln Met Glu Asp Pro Thr Phe Lys Glu Asn Tyr Arg Phe His Ala

1905                1910                1915                1920

Ile Asn Gly Tyr Ile Met Asp Thr Leu Pro Gly Leu Val Met Ala Gln
                1925                1930                1935

Asp Gln Arg Ile Arg Trp Tyr Leu Leu Ser Met Gly Ser Asn Glu Asn
            1940                1945                1950

Ile His Ser Ile His Phe Ser Gly His Val Phe Thr Val Arg Lys Lys
        1955                1960                1965

Glu Glu Tyr Lys Met Ala Leu Tyr Asn Leu Tyr Pro Gly Val Phe Glu
    1970                1975                1980

Thr Val Glu Met Leu Pro Ser Lys Ala Gly Ile Trp Arg Val Glu Cys
1985                1990                1995                2000

Leu Ile Gly Glu His Leu His Ala Gly Met Ser Thr Leu Phe Leu Val
                2005                2010                2015

Tyr Ser Asn Lys Cys Gln Thr Pro Leu Gly Met Ala Ser Gly His Ile
            2020                2025                2030

Arg Asp Phe Gln Ile Thr Ala Ser Gly Gln Tyr Gly Gln Trp Ala Pro
        2035                2040                2045

Lys Leu Ala Arg Leu His Tyr Ser Gly Ser Ile Asn Ala Trp Ser Thr
    2050                2055                2060

Lys Glu Pro Phe Ser Trp Ile Lys Val Asp Leu Leu Ala Pro Met Ile
2065                2070                2075                2080

Ile His Gly Ile Lys Thr Gln Gly Ala Arg Gln Lys Phe Ser Ser Leu
                2085                2090                2095

Tyr Ile Ser Gln Phe Ile Ile Met Tyr Ser Leu Asp Gly Lys Lys Trp
            2100                2105                2110

Gln Thr Tyr Arg Gly Asn Ser Thr Gly Thr Leu Met Val Phe Phe Gly
        2115                2120                2125

Asn Val Asp Ser Ser Gly Ile Lys His Asn Ile Phe Asn Pro Pro Ile
    2130                2135                2140

Ile Ala Arg Tyr Ile Arg Leu His Pro Thr His Tyr Ser Ile Arg Ser
2145                2150                2155                2160

Thr Leu Arg Met Glu Leu Met Gly Cys Asp Leu Asn Ser Cys Ser Met
                2165                2170                2175

Pro Leu Gly Met Glu Ser Lys Ala Ile Ser Asp Ala Gln Ile Thr Ala
            2180                2185                2190

Ser Ser Tyr Phe Thr Asn Met Phe Ala Thr Trp Ser Pro Ser Lys Ala
        2195                2200                2205

Arg Leu His Leu Gln Gly Arg Ser Asn Ala Trp Arg Pro Gln Val Asn
    2210                2215                2220

Asn Pro Lys Glu Trp Leu Gln Val Asp Phe Gln Lys Thr Met Lys Val
2225                2230                2235                2240

Thr Gly Val Thr Thr Gln Gly Val Lys Ser Leu Leu Thr Ser Met Tyr

2245                2250                2255

Val Lys Glu Phe Leu Ile Ser Ser Ser Gln Asp Gly His Gln Trp Thr
        2260                2265                2270

Leu Phe Phe Gln Asn Gly Lys Val Lys Val Phe Gln Gly Asn Gln Asp
    2275                2280                2285

Ser Phe Thr Pro Val Val Asn Ser Leu Asp Pro Pro Leu Leu Thr Arg
  2290                2295                2300

Tyr Leu Arg Ile His Pro Gln Ser Trp Val His Gln Ile Ala Leu Arg
2305                2310                2315                2320

Met Glu Val Leu Gly Cys Glu Ala Gln Asp Leu Tyr
                2325                2330

<210>    2
<211>    1467
<212>    PRT
<213>    Artificial Sequence

<220>
<223>    G2 (scF2)

<400>    2
Ala Thr Arg Arg Tyr Tyr Leu Gly Ala Val Glu Leu Ser Trp Asp Tyr
 1               5                  10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Thr Leu Gln Ser Asp Gln Glu Glu Ile Asp Tyr Asp
785                 790                 795                 800

Asp Thr Ile Ser Val Glu Met Lys Lys Glu Asp Phe Asp Ile Tyr Asp
                805                 810                 815

Glu Asp Glu Asn Gln Ser Pro Arg Ser Phe Gln Lys Lys Thr Arg His
            820                 825                 830

Tyr Phe Ile Ala Ala Val Glu Arg Leu Trp Asp Tyr Gly Met Ser Ser
        835                 840                 845

Ser Pro His Val Leu Arg Asn Arg Ala Gln Ser Gly Ser Val Pro Gln
    850                 855                 860

Phe Lys Lys Val Val Phe Gln Glu Phe Thr Asp Gly Ser Phe Thr Gln
865                 870                 875                 880

Pro Leu Tyr Arg Gly Glu Leu Asn Glu His Leu Gly Leu Leu Gly Pro
                885                 890                 895

Tyr Ile Arg Ala Glu Val Glu Asp Asn Ile Met Val Thr Phe Arg Asn
            900                 905                 910

Gln Ala Ser Arg Pro Tyr Ser Phe Tyr Ser Ser Leu Ile Ser Tyr Glu
        915                 920                 925

Glu Asp Gln Arg Gln Gly Ala Glu Pro Arg Lys Asn Phe Val Lys Pro
    930                 935                 940

Asn Glu Thr Lys Thr Tyr Phe Trp Lys Val Gln His His Met Ala Pro
945                 950                 955                 960

Thr Lys Asp Glu Phe Asp Cys Lys Ala Trp Ala Tyr Phe Ser Asp Val
                965                 970                 975

Asp Leu Glu Lys Asp Val His Ser Gly Leu Ile Gly Pro Leu Leu Val
            980                 985                 990

Cys His Thr Asn Thr Leu Asn Pro Ala His Gly Arg Gln Val Thr Val
        995                 1000                1005

Gln Glu Phe Ala Leu Phe Phe Thr Ile Phe Asp Glu Thr Lys Ser Trp
    1010                1015                1020

Tyr Phe Thr Glu Asn Met Glu Arg Asn Cys Arg Ala Pro Cys Asn Ile
1025                1030                1035                1040

Gln Met Glu Asp Pro Thr Phe Lys Glu Asn Tyr Arg Phe His Ala Ile
                1045                1050                1055

Asn Gly Tyr Ile Met Asp Thr Leu Pro Gly Leu Val Met Ala Gln Asp
            1060                1065                1070

Gln Arg Ile Arg Trp Tyr Leu Leu Ser Met Gly Ser Asn Glu Asn Ile
        1075                1080                1085

His Ser Ile His Phe Ser Gly His Val Phe Thr Val Arg Lys Lys Glu
    1090                1095                1100

Glu Tyr Lys Met Ala Leu Tyr Asn Leu Tyr Pro Gly Val Phe Glu Thr
1105                1110                1115                1120

Val Glu Met Leu Pro Ser Lys Ala Gly Ile Trp Arg Val Glu Cys Leu
                1125                1130                1135

Ile Gly Glu His Leu His Ala Gly Met Ser Thr Leu Phe Leu Val Tyr
            1140                1145                1150

Ser Asn Lys Cys Gln Thr Pro Leu Gly Met Ala Ser Gly His Ile Arg
        1155                1160                1165

Asp Phe Gln Ile Thr Ala Ser Gly Gln Tyr Gly Gln Trp Ala Pro Lys
    1170                1175                1180

Leu Ala Arg Leu His Tyr Ser Gly Ser Ile Asn Ala Trp Ser Thr Lys
1185                1190                1195                1200

Glu Pro Phe Ser Trp Ile Lys Val Asp Leu Leu Ala Pro Met Ile Ile
                1205                1210                1215

His Gly Ile Lys Thr Gln Gly Ala Arg Gln Lys Phe Ser Ser Leu Tyr
            1220                1225                1230

Ile Ser Gln Phe Ile Ile Met Tyr Ser Leu Asp Gly Lys Lys Trp Gln
        1235                1240                1245

Thr Tyr Arg Gly Asn Ser Thr Gly Thr Leu Met Val Phe Phe Gly Asn
    1250                1255                1260

Val Asp Ser Ser Gly Ile Lys His Asn Ile Phe Asn Pro Pro Ile Ile
1265                1270                1275                1280

Ala Arg Tyr Ile Arg Leu His Pro Thr His Tyr Ser Ile Arg Ser Thr
                1285                1290                1295

Leu Arg Met Glu Leu Met Gly Cys Asp Leu Asn Ser Cys Ser Met Pro
            1300                1305                1310

Leu Gly Met Glu Ser Lys Ala Ile Ser Asp Ala Gln Ile Thr Ala Ser
        1315                1320                1325

Ser Tyr Phe Thr Asn Met Phe Ala Thr Trp Ser Pro Ser Lys Ala Arg
    1330                1335                1340

Leu His Leu Gln Gly Arg Ser Asn Ala Trp Arg Pro Gln Val Asn Asn
1345                1350                1355                1360

Pro Lys Glu Trp Leu Gln Val Asp Phe Gln Lys Thr Met Lys Val Thr
                1365                1370                1375

Gly Val Thr Thr Gln Gly Val Lys Ser Leu Leu Thr Ser Met Tyr Val
            1380                1385                1390

Lys Glu Phe Leu Ile Ser Ser Ser Gln Asp Gly His Gln Trp Thr Leu
        1395                1400                1405

Phe Phe Gln Asn Gly Lys Val Lys Val Phe Gln Gly Asn Gln Asp Ser
    1410                1415                1420

Phe Thr Pro Val Val Asn Ser Leu Asp Pro Pro Leu Leu Thr Arg Tyr
1425                1430                1435                1440

Leu Arg Ile His Pro Gln Ser Trp Val His Gln Ile Ala Leu Arg Met
                1445                1450                1455

Glu Val Leu Gly Cys Glu Ala Gln Asp Leu Tyr
            1460                1465


<210>    3
<211>    1511
<212>    PRT
<213>    Artificial Sequence

<220>
<223>    G3 (scF3)


<400>    3

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350
Ser Glu Met Asp Val Val Arg Phe Asp Asp Asp Asn Ser Pro Ser Phe
        355                 360                 365
Ile Gln Ile Arg Ser Val Ala Lys Lys His Pro Lys Thr Trp Val His
    370                 375                 380
Tyr Ile Ala Ala Glu Glu Glu Asp Trp Asp Tyr Ala Pro Leu Val Leu
385                 390                 395                 400
Ala Pro Asp Asp Arg Ser Tyr Lys Ser Gln Tyr Leu Asn Asn Gly Pro
                405                 410                 415
Gln Arg Ile Gly Arg Lys Tyr Lys Lys Val Arg Phe Met Ala Tyr Thr
            420                 425                 430
Asp Glu Thr Phe Lys Thr Arg Glu Ala Ile Gln His Glu Ser Gly Ile
        435                 440                 445
Leu Gly Pro Leu Leu Tyr Gly Glu Val Gly Asp Thr Leu Leu Ile Ile
    450                 455                 460
Phe Lys Asn Gln Ala Ser Arg Pro Tyr Asn Ile Tyr Pro His Gly Ile
465                 470                 475                 480
Thr Asp Val Arg Pro Leu Tyr Ser Arg Arg Leu Pro Lys Gly Val Lys
                485                 490                 495
His Leu Lys Asp Phe Pro Ile Leu Pro Gly Glu Ile Phe Lys Tyr Lys
            500                 505                 510
Trp Thr Val Thr Val Glu Asp Gly Pro Thr Lys Ser Asp Pro Arg Cys
        515                 520                 525
Leu Thr Arg Tyr Tyr Ser Ser Phe Val Asn Met Glu Arg Asp Leu Ala
    530                 535                 540
Ser Gly Leu Ile Gly Pro Leu Leu Ile Cys Tyr Lys Glu Ser Val Asp
545                 550                 555                 560
Gln Arg Gly Asn Gln Ile Met Ser Asp Lys Arg Asn Val Ile Leu Phe
                565                 570                 575
Ser Val Phe Asp Glu Asn Arg Ser Trp Tyr Leu Thr Glu Asn Ile Gln
            580                 585                 590
Arg Phe Leu Pro Asn Pro Ala Gly Val Gln Leu Glu Asp Pro Glu Phe
        595                 600                 605
Gln Ala Ser Asn Ile Met His Ser Ile Asn Gly Tyr Val Phe Asp Ser
    610                 615                 620
Leu Gln Leu Ser Val Cys Leu His Glu Val Ala Tyr Trp Tyr Ile Leu
625                 630                 635                 640
Ser Ile Gly Ala Gln Thr Asp Phe Leu Ser Val Phe Phe Ser Gly Tyr
                645                 650                 655
Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Thr Leu Gln Ser Asp Gln Glu Glu Ile Asp Tyr Asp Asp Thr Ile Ser
        835                 840                 845

Val Glu Met Lys Lys Glu Asp Phe Asp Ile Tyr Asp Glu Asp Glu Asn
    850                 855                 860

Gln Ser Pro Arg Ser Phe Gln Lys Lys Thr Arg His Tyr Phe Ile Ala
865                 870                 875                 880

Ala Val Glu Arg Leu Trp Asp Tyr Gly Met Ser Ser Ser Pro His Val
                885                 890                 895

Leu Arg Asn Arg Ala Gln Ser Gly Ser Val Pro Gln Phe Lys Lys Val
            900                 905                 910

Val Phe Gln Glu Phe Thr Asp Gly Ser Phe Thr Gln Pro Leu Tyr Arg
        915                 920                 925

Gly Glu Leu Asn Glu His Leu Gly Leu Leu Gly Pro Tyr Ile Arg Ala
    930                 935                 940

Glu Val Glu Asp Asn Ile Met Val Thr Phe Arg Asn Gln Ala Ser Arg
945                 950                 955                 960

Pro Tyr Ser Phe Tyr Ser Ser Leu Ile Ser Tyr Glu Glu Asp Gln Arg
                965                 970                 975

Gln Gly Ala Glu Pro Arg Lys Asn Phe Val Lys Pro Asn Glu Thr Lys
            980                 985                 990

Thr Tyr Phe Trp Lys Val Gln His His Met Ala Pro Thr Lys Asp Glu
        995                 1000                1005

Phe Asp Cys Lys Ala Trp Ala Tyr Phe Ser Asp Val Asp Leu Glu Lys
    1010                1015                1020

Asp Val His Ser Gly Leu Ile Gly Pro Leu Leu Val Cys His Thr Asn
1025                1030                1035                1040

Thr Leu Asn Pro Ala His Gly Arg Gln Val Thr Val Gln Glu Phe Ala
                1045                1050                1055

Leu Phe Phe Thr Ile Phe Asp Glu Thr Lys Ser Trp Tyr Phe Thr Glu
            1060                1065                1070

Asn Met Glu Arg Asn Cys Arg Ala Pro Cys Asn Ile Gln Met Glu Asp
        1075                1080                1085

Pro Thr Phe Lys Glu Asn Tyr Arg Phe His Ala Ile Asn Gly Tyr Ile
    1090                1095                1100

Met Asp Thr Leu Pro Gly Leu Val Met Ala Gln Asp Gln Arg Ile Arg
1105                1110                1115                1120

Trp Tyr Leu Leu Ser Met Gly Ser Asn Glu Asn Ile His Ser Ile His
                1125                1130                1135

Phe Ser Gly His Val Phe Thr Val Arg Lys Lys Glu Glu Tyr Lys Met
            1140                1145                1150

Ala Leu Tyr Asn Leu Tyr Pro Gly Val Phe Glu Thr Val Glu Met Leu
        1155                1160                1165

Pro Ser Lys Ala Gly Ile Trp Arg Val Glu Cys Leu Ile Gly Glu His
    1170                1175                1180

Leu His Ala Gly Met Ser Thr Leu Phe Leu Val Tyr Ser Asn Lys Cys
1185                1190                1195                1200

Gln Thr Pro Leu Gly Met Ala Ser Gly His Ile Arg Asp Phe Gln Ile
                1205                1210                1215

Thr Ala Ser Gly Gln Tyr Gly Gln Trp Ala Pro Lys Leu Ala Arg Leu
            1220                1225                1230

His Tyr Ser Gly Ser Ile Asn Ala Trp Ser Thr Lys Glu Pro Phe Ser
        1235                1240                1245

Trp Ile Lys Val Asp Leu Leu Ala Pro Met Ile Ile His Gly Ile Lys
    1250                1255                1260

Thr Gln Gly Ala Arg Gln Lys Phe Ser Ser Leu Tyr Ile Ser Gln Phe
1265                1270                1275                1280

Ile Ile Met Tyr Ser Leu Asp Gly Lys Lys Trp Gln Thr Tyr Arg Gly
                1285                1290                1295

Asn Ser Thr Gly Thr Leu Met Val Phe Phe Gly Asn Val Asp Ser Ser
            1300                1305                1310

Gly Ile Lys His Asn Ile Phe Asn Pro Pro Ile Ile Ala Arg Tyr Ile
        1315                1320                1325

Arg Leu His Pro Thr His Tyr Ser Ile Arg Ser Thr Leu Arg Met Glu
    1330                1335                1340

Leu Met Gly Cys Asp Leu Asn Ser Cys Ser Met Pro Leu Gly Met Glu
1345                1350                1355                1360

Ser Lys Ala Ile Ser Asp Ala Gln Ile Thr Ala Ser Ser Tyr Phe Thr
                1365                1370                1375

Asn Met Phe Ala Thr Trp Ser Pro Ser Lys Ala Arg Leu His Leu Gln
            1380                1385                1390

Gly Arg Ser Asn Ala Trp Arg Pro Gln Val Asn Asn Pro Lys Glu Trp
        1395                1400                1405

Leu Gln Val Asp Phe Gln Lys Thr Met Lys Val Thr Gly Val Thr Thr
    1410                1415                1420

Gln Gly Val Lys Ser Leu Leu Thr Ser Met Tyr Val Lys Glu Phe Leu
1425                1430                1435                1440

Ile Ser Ser Ser Gln Asp Gly His Gln Trp Thr Leu Phe Phe Gln Asn
                1445                1450                1455

Gly Lys Val Lys Val Phe Gln Gly Asn Gln Asp Ser Phe Thr Pro Val
            1460                1465                1470

Val Asn Ser Leu Asp Pro Pro Leu Leu Thr Arg Tyr Leu Arg Ile His
        1475                1480                1485

Pro Gln Ser Trp Val His Gln Ile Ala Leu Arg Met Glu Val Leu Gly
    1490                1495                1500

Cys Glu Ala Gln Asp Leu Tyr
1505                1510


<210>    4
<211>    1581
<212>    PRT
<213>    Artificial Sequence

<220>
<223>    G4 (scF4)


<400>    4
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala

            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile

435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn

770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val

1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys

1445                1450                1455

Ala Arg Leu His Leu Gln Gly Arg Ser Asn Ala Trp Arg Pro Gln Val
        1460                1465                1470

Asn Asn Pro Lys Glu Trp Leu Gln Val Asp Phe Gln Lys Thr Met Lys
    1475                1480                1485

Val Thr Gly Val Thr Thr Gln Gly Val Lys Ser Leu Leu Thr Ser Met
    1490                1495                1500

Tyr Val Lys Glu Phe Leu Ile Ser Ser Ser Gln Asp Gly His Gln Trp
1505                1510                1515                1520

Thr Leu Phe Phe Gln Asn Gly Lys Val Lys Val Phe Gln Gly Asn Gln
                1525                1530                1535

Asp Ser Phe Thr Pro Val Val Asn Ser Leu Asp Pro Pro Leu Leu Thr
        1540                1545                1550

Arg Tyr Leu Arg Ile His Pro Gln Ser Trp Val His Gln Ile Ala Leu
    1555                1560                1565

Arg Met Glu Val Leu Gly Cys Glu Ala Gln Asp Leu Tyr
    1570                1575                1580

<210>    5
<211>    1644
<212>    PRT
<213>    Artificial Sequence

<220>
<223>    G6 (scF5)

<400>    5
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn

130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile

465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe

805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Thr Leu Gln Ser Asp Gln Glu Glu Ile Asp Tyr
                965                 970                 975

Asp Asp Thr Ile Ser Val Glu Met Lys Lys Glu Asp Phe Asp Ile Tyr
            980                 985                 990

Asp Glu Asp Glu Asn Gln Ser Pro Arg Ser Phe Gln Lys Lys Thr Arg
        995                 1000                1005

His Tyr Phe Ile Ala Ala Val Glu Arg Leu Trp Asp Tyr Gly Met Ser
    1010                1015                1020

Ser Ser Pro His Val Leu Arg Asn Arg Ala Gln Ser Gly Ser Val Pro
1025                1030                1035                1040

Gln Phe Lys Lys Val Val Phe Gln Glu Phe Thr Asp Gly Ser Phe Thr
                1045                1050                1055

Gln Pro Leu Tyr Arg Gly Glu Leu Asn Glu His Leu Gly Leu Leu Gly
            1060                1065                1070

Pro Tyr Ile Arg Ala Glu Val Glu Asp Asn Ile Met Val Thr Phe Arg
        1075                1080                1085

Asn Gln Ala Ser Arg Pro Tyr Ser Phe Tyr Ser Ser Leu Ile Ser Tyr
    1090                1095                1100

Glu Glu Asp Gln Arg Gln Gly Ala Glu Pro Arg Lys Asn Phe Val Lys
1105                1110                1115                1120

Pro Asn Glu Thr Lys Thr Tyr Phe Trp Lys Val Gln His His Met Ala
                1125                1130                1135

Pro Thr Lys Asp Glu Phe Asp Cys Lys Ala Trp Ala Tyr Phe Ser Asp

1140                1145                1150

Val Asp Leu Glu Lys Asp Val His Ser Gly Leu Ile Gly Pro Leu Leu
        1155                1160                1165

Val Cys His Thr Asn Thr Leu Asn Pro Ala His Gly Arg Gln Val Thr
    1170                1175                1180

Val Gln Glu Phe Ala Leu Phe Phe Thr Ile Phe Asp Glu Thr Lys Ser
1185                1190                1195                1200

Trp Tyr Phe Thr Glu Asn Met Glu Arg Asn Cys Arg Ala Pro Cys Asn
                1205                1210                1215

Ile Gln Met Glu Asp Pro Thr Phe Lys Glu Asn Tyr Arg Phe His Ala
            1220                1225                1230

Ile Asn Gly Tyr Ile Met Asp Thr Leu Pro Gly Leu Val Met Ala Gln
        1235                1240                1245

Asp Gln Arg Ile Arg Trp Tyr Leu Leu Ser Met Gly Ser Asn Glu Asn
    1250                1255                1260

Ile His Ser Ile His Phe Ser Gly His Val Phe Thr Val Arg Lys Lys
1265                1270                1275                1280

Glu Glu Tyr Lys Met Ala Leu Tyr Asn Leu Tyr Pro Gly Val Phe Glu
                1285                1290                1295

Thr Val Glu Met Leu Pro Ser Lys Ala Gly Ile Trp Arg Val Glu Cys
            1300                1305                1310

Leu Ile Gly Glu His Leu His Ala Gly Met Ser Thr Leu Phe Leu Val
        1315                1320                1325

Tyr Ser Asn Lys Cys Gln Thr Pro Leu Gly Met Ala Ser Gly His Ile
    1330                1335                1340

Arg Asp Phe Gln Ile Thr Ala Ser Gly Gln Tyr Gly Gln Trp Ala Pro
1345                1350                1355                1360

Lys Leu Ala Arg Leu His Tyr Ser Gly Ser Ile Asn Ala Trp Ser Thr
                1365                1370                1375

Lys Glu Pro Phe Ser Trp Ile Lys Val Asp Leu Leu Ala Pro Met Ile
            1380                1385                1390

Ile His Gly Ile Lys Thr Gln Gly Ala Arg Gln Lys Phe Ser Ser Leu
        1395                1400                1405

Tyr Ile Ser Gln Phe Ile Ile Met Tyr Ser Leu Asp Gly Lys Lys Trp
    1410                1415                1420

Gln Thr Tyr Arg Gly Asn Ser Thr Gly Thr Leu Met Val Phe Phe Gly
1425                1430                1435                1440

Asn Val Asp Ser Ser Gly Ile Lys His Asn Ile Phe Asn Pro Pro Ile
                1445                1450                1455

Ile Ala Arg Tyr Ile Arg Leu His Pro Thr His Tyr Ser Ile Arg Ser
            1460                1465                1470

Thr Leu Arg Met Glu Leu Met Gly Cys Asp Leu Asn Ser Cys Ser Met

1475                1480                1485

Pro Leu Gly Met Glu Ser Lys Ala Ile Ser Asp Ala Gln Ile Thr Ala
    1490                1495                1500

Ser Ser Tyr Phe Thr Asn Met Phe Ala Thr Trp Ser Pro Ser Lys Ala
1505                1510                1515                1520

Arg Leu His Leu Gln Gly Arg Ser Asn Ala Trp Arg Pro Gln Val Asn
                1525                1530                1535

Asn Pro Lys Glu Trp Leu Gln Val Asp Phe Gln Lys Thr Met Lys Val
            1540                1545                1550

Thr Gly Val Thr Thr Gln Gly Val Lys Ser Leu Leu Thr Ser Met Tyr
        1555                1560                1565

Val Lys Glu Phe Leu Ile Ser Ser Ser Gln Asp Gly His Gln Trp Thr
    1570                1575                1580

Leu Phe Phe Gln Asn Gly Lys Val Lys Val Phe Gln Gly Asn Gln Asp
1585                1590                1595                1600

Ser Phe Thr Pro Val Val Asn Ser Leu Asp Pro Pro Leu Leu Thr Arg
                1605                1610                1615

Tyr Leu Arg Ile His Pro Gln Ser Trp Val His Gln Ile Ala Leu Arg
            1620                1625                1630

Met Glu Val Leu Gly Cys Glu Ala Gln Asp Leu Tyr
        1635                1640

<210>   6
<211>   1587
<212>   PRT
<213>   Artificial Sequence

<220>
<223>   G4_flex (scF6)

<400>   6
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Gln Asn Pro Pro Val Thr Leu Gln Ser
            900                 905                 910

Asp Gln Glu Glu Ile Asp Tyr Asp Asp Thr Ile Ser Val Glu Met Lys
        915                 920                 925

Lys Glu Asp Phe Asp Ile Tyr Asp Glu Asp Glu Asn Gln Ser Pro Arg
    930                 935                 940

Ser Phe Gln Lys Lys Thr Arg His Tyr Phe Ile Ala Ala Val Glu Arg
945                 950                 955                 960

Leu Trp Asp Tyr Gly Met Ser Ser Ser Pro His Val Leu Arg Asn Arg
                965                 970                 975

Ala Gln Ser Gly Ser Val Pro Gln Phe Lys Lys Val Val Phe Gln Glu
            980                 985                 990

Phe Thr Asp Gly Ser Phe Thr Gln Pro Leu Tyr Arg Gly Glu Leu Asn
        995                 1000                1005

Glu His Leu Gly Leu Leu Gly Pro Tyr Ile Arg Ala Glu Val Glu Asp
    1010                1015                1020

Asn Ile Met Val Thr Phe Arg Asn Gln Ala Ser Arg Pro Tyr Ser Phe
1025                1030                1035                1040

Tyr Ser Ser Leu Ile Ser Tyr Glu Glu Asp Gln Arg Gln Gly Ala Glu
                1045                1050                1055

Pro Arg Lys Asn Phe Val Lys Pro Asn Glu Thr Lys Thr Tyr Phe Trp
            1060                1065                1070

Lys Val Gln His His Met Ala Pro Thr Lys Asp Glu Phe Asp Cys Lys
        1075                1080                1085

Ala Trp Ala Tyr Phe Ser Asp Val Asp Leu Glu Lys Asp Val His Ser
    1090                1095                1100

Gly Leu Ile Gly Pro Leu Leu Val Cys His Thr Asn Thr Leu Asn Pro
1105                1110                1115                1120

Ala His Gly Arg Gln Val Thr Val Gln Glu Phe Ala Leu Phe Phe Thr
                1125                1130                1135

Ile Phe Asp Glu Thr Lys Ser Trp Tyr Phe Thr Glu Asn Met Glu Arg
            1140                1145                1150

Asn Cys Arg Ala Pro Cys Asn Ile Gln Met Glu Asp Pro Thr Phe Lys
        1155                1160                1165

Glu Asn Tyr Arg Phe His Ala Ile Asn Gly Tyr Ile Met Asp Thr Leu
    1170                1175                1180

Pro Gly Leu Val Met Ala Gln Asp Gln Arg Ile Arg Trp Tyr Leu Leu
1185                1190                1195                1200

Ser Met Gly Ser Asn Glu Asn Ile His Ser Ile His Phe Ser Gly His
                1205                1210                1215

Val Phe Thr Val Arg Lys Lys Glu Glu Tyr Lys Met Ala Leu Tyr Asn
            1220                1225                1230

Leu Tyr Pro Gly Val Phe Glu Thr Val Glu Met Leu Pro Ser Lys Ala
        1235                1240                1245

Gly Ile Trp Arg Val Glu Cys Leu Ile Gly Glu His Leu His Ala Gly
    1250                1255                1260

Met Ser Thr Leu Phe Leu Val Tyr Ser Asn Lys Cys Gln Thr Pro Leu
1265                1270                1275                1280

Gly Met Ala Ser Gly His Ile Arg Asp Phe Gln Ile Thr Ala Ser Gly
                1285                1290                1295

Gln Tyr Gly Gln Trp Ala Pro Lys Leu Ala Arg Leu His Tyr Ser Gly
            1300                1305                1310

Ser Ile Asn Ala Trp Ser Thr Lys Glu Pro Phe Ser Trp Ile Lys Val
        1315                1320                1325

Asp Leu Leu Ala Pro Met Ile Ile His Gly Ile Lys Thr Gln Gly Ala
    1330                1335                1340

Arg Gln Lys Phe Ser Ser Leu Tyr Ile Ser Gln Phe Ile Ile Met Tyr
1345                1350                1355                1360

Ser Leu Asp Gly Lys Lys Trp Gln Thr Tyr Arg Gly Asn Ser Thr Gly
                1365                1370                1375

Thr Leu Met Val Phe Phe Gly Asn Val Asp Ser Ser Gly Ile Lys His
            1380                1385                1390

Asn Ile Phe Asn Pro Pro Ile Ile Ala Arg Tyr Ile Arg Leu His Pro
        1395                1400                1405

Thr His Tyr Ser Ile Arg Ser Thr Leu Arg Met Glu Leu Met Gly Cys
    1410                1415                1420

Asp Leu Asn Ser Cys Ser Met Pro Leu Gly Met Glu Ser Lys Ala Ile
1425                1430                1435                1440

Ser Asp Ala Gln Ile Thr Ala Ser Ser Tyr Phe Thr Asn Met Phe Ala
                1445                1450                1455

Thr Trp Ser Pro Ser Lys Ala Arg Leu His Leu Gln Gly Arg Ser Asn
            1460                1465                1470

Ala Trp Arg Pro Gln Val Asn Asn Pro Lys Glu Trp Leu Gln Val Asp
        1475                1480                1485

Phe Gln Lys Thr Met Lys Val Thr Gly Val Thr Thr Gln Gly Val Lys
    1490                1495                1500

Ser Leu Leu Thr Ser Met Tyr Val Lys Glu Phe Leu Ile Ser Ser Ser
1505                1510                1515                1520

Gln Asp Gly His Gln Trp Thr Leu Phe Phe Gln Asn Gly Lys Val Lys
                1525                1530                1535

Val Phe Gln Gly Asn Gln Asp Ser Phe Thr Pro Val Val Asn Ser Leu
            1540                1545                1550

Asp Pro Pro Leu Leu Thr Arg Tyr Leu Arg Ile His Pro Gln Ser Trp
        1555                1560                1565

Val His Gln Ile Ala Leu Arg Met Glu Val Leu Gly Cys Glu Ala Gln
    1570                1575                1580

Asp Leu Tyr
1585

<210>    7
<211>    1644
<212>    PRT
<213>    Artificial Sequence

<220>
<223>    G6_opt (scF7)

<400>    7
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                    805                 810                 815

Ser Asp Asp Pro Ser Pro Gly Ala Ile Asp Ser Asn Asn Ser Leu Ser
                820                 825                 830

Glu Met Thr His Phe Arg Pro Gln Leu His His Ser Gly Asp Met Val
            835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Thr Leu Gln Ser Asp Gln Glu Glu Ile Asp Tyr
                965                 970                 975

Asp Asp Thr Ile Ser Val Glu Met Lys Lys Glu Asp Phe Asp Ile Tyr
            980                 985                 990

Asp Glu Asp Glu Asn Gln Ser Pro Arg Ser Phe Gln Lys Lys Thr Arg
        995                 1000                1005

His Tyr Phe Ile Ala Ala Val Glu Arg Leu Trp Asp Tyr Gly Met Ser
    1010                1015                1020

Ser Ser Pro His Val Leu Arg Asn Arg Ala Gln Ser Gly Ser Val Pro
1025                1030                1035                1040

Gln Phe Lys Lys Val Val Phe Gln Glu Phe Thr Asp Gly Ser Phe Thr
                1045                1050                1055

Gln Pro Leu Tyr Arg Gly Glu Leu Asn Glu His Leu Gly Leu Leu Gly
            1060                1065                1070

Pro Tyr Ile Arg Ala Glu Val Glu Asp Asn Ile Met Val Thr Phe Arg
        1075                1080                1085

Asn Gln Ala Ser Arg Pro Tyr Ser Phe Tyr Ser Ser Leu Ile Ser Tyr
    1090                1095                1100

Glu Glu Asp Gln Arg Gln Gly Ala Glu Pro Arg Lys Asn Phe Val Lys
1105                1110                1115                1120

Pro Asn Glu Thr Lys Thr Tyr Phe Trp Lys Val Gln His His Met Ala
                1125                1130                1135

Pro Thr Lys Asp Glu Phe Asp Cys Lys Ala Trp Ala Tyr Phe Ser Asp
            1140                1145                1150

Val Asp Leu Glu Lys Asp Val His Ser Gly Leu Ile Gly Pro Leu Leu
        1155                1160                1165

Val Cys His Thr Asn Thr Leu Asn Pro Ala His Gly Arg Gln Val Thr
    1170                1175                1180

Val Gln Glu Phe Ala Leu Phe Phe Thr Ile Phe Asp Glu Thr Lys Ser
1185                1190                1195                1200

Trp Tyr Phe Thr Glu Asn Met Glu Arg Asn Cys Arg Ala Pro Cys Asn
                1205                1210                1215

Ile Gln Met Glu Asp Pro Thr Phe Lys Glu Asn Tyr Arg Phe His Ala
            1220                1225                1230

Ile Asn Gly Tyr Ile Met Asp Thr Leu Pro Gly Leu Val Met Ala Gln
        1235                1240                1245

Asp Gln Arg Ile Arg Trp Tyr Leu Leu Ser Met Gly Ser Asn Glu Asn
    1250                1255                1260

Ile His Ser Ile His Phe Ser Gly His Val Phe Thr Val Arg Lys Lys
1265                1270                1275                1280

Glu Glu Tyr Lys Met Ala Leu Tyr Asn Leu Tyr Pro Gly Val Phe Glu
                1285                1290                1295

Thr Val Glu Met Leu Pro Ser Lys Ala Gly Ile Trp Arg Val Glu Cys
            1300                1305                1310

Leu Ile Gly Glu His Leu His Ala Gly Met Ser Thr Leu Phe Leu Val
        1315                1320                1325

Tyr Ser Asn Lys Cys Gln Thr Pro Leu Gly Met Ala Ser Gly His Ile
    1330                1335                1340

Arg Asp Phe Gln Ile Thr Ala Ser Gly Gln Tyr Gly Gln Trp Ala Pro
1345                1350                1355                1360

Lys Leu Ala Arg Leu His Tyr Ser Gly Ser Ile Asn Ala Trp Ser Thr
                1365                1370                1375

Lys Glu Pro Phe Ser Trp Ile Lys Val Asp Leu Leu Ala Pro Met Ile
            1380                1385                1390

Ile His Gly Ile Lys Thr Gln Gly Ala Arg Gln Lys Phe Ser Ser Leu
        1395                1400                1405

Tyr Ile Ser Gln Phe Ile Ile Met Tyr Ser Leu Asp Gly Lys Lys Trp
    1410                1415                1420

Gln Thr Tyr Arg Gly Asn Ser Thr Gly Thr Leu Met Val Phe Phe Gly
1425                1430                1435                1440

Asn Val Asp Ser Ser Gly Ile Lys His Asn Ile Phe Asn Pro Pro Ile
                1445                1450                1455

Ile Ala Arg Tyr Ile Arg Leu His Pro Thr His Tyr Ser Ile Arg Ser
            1460                1465                1470

Thr Leu Arg Met Glu Leu Met Gly Cys Asp Leu Asn Ser Cys Ser Met
        1475                1480                1485

Pro Leu Gly Met Glu Ser Lys Ala Ile Ser Asp Ala Gln Ile Thr Ala
    1490                1495                1500

Ser Ser Tyr Phe Thr Asn Met Phe Ala Thr Trp Ser Pro Ser Lys Ala
1505                1510                1515                1520

Arg Leu His Leu Gln Gly Arg Ser Asn Ala Trp Arg Pro Gln Val Asn
                1525                1530                1535

Asn Pro Lys Glu Trp Leu Gln Val Asp Phe Gln Lys Thr Met Lys Val
            1540                1545                1550

Thr Gly Val Thr Thr Gln Gly Val Lys Ser Leu Leu Thr Ser Met Tyr
        1555                1560                1565

Val Lys Glu Phe Leu Ile Ser Ser Ser Gln Asp Gly His Gln Trp Thr
    1570                1575                1580

Leu Phe Phe Gln Asn Gly Lys Val Lys Val Phe Gln Gly Asn Gln Asp
1585                1590                1595                1600

Ser Phe Thr Pro Val Val Asn Ser Leu Asp Pro Pro Leu Leu Thr Arg
                1605                1610                1615

Tyr Leu Arg Ile His Pro Gln Ser Trp Val His Gln Ile Ala Leu Arg
            1620                1625                1630

Met Glu Val Leu Gly Cys Glu Ala Gln Asp Leu Tyr
        1635                1640


<210>    8
<211>    1581
<212>    PRT
<213>    Artificial Sequence

<220>
<223>    G4_B3


<400>    8
Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala

            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile

        435                 440                 445

Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Pro Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Cys Gln Asn

770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Thr Leu Gln Ser Asp Gln Glu Glu Ile Asp
            900                 905                 910

Tyr Asp Asp Thr Ile Ser Val Glu Met Lys Lys Glu Asp Phe Asp Ile
        915                 920                 925

Tyr Asp Glu Asp Glu Asn Gln Ser Pro Arg Ser Phe Gln Lys Lys Thr
    930                 935                 940

Arg His Tyr Phe Ile Ala Ala Val Glu Arg Leu Trp Asp Tyr Gly Met
945                 950                 955                 960

Ser Ser Ser Pro His Val Leu Arg Asn Arg Ala Gln Ser Gly Ser Val
                965                 970                 975

Pro Gln Phe Lys Lys Val Val Phe Gln Glu Phe Thr Asp Gly Ser Phe
            980                 985                 990

Thr Gln Pro Leu Tyr Arg Gly Glu Leu Asn Glu His Leu Gly Leu Leu
        995                 1000                1005

Gly Pro Tyr Ile Arg Ala Glu Val Glu Asp Asn Ile Met Val Thr Phe
    1010                1015                1020

Arg Asn Gln Ala Ser Arg Pro Tyr Ser Phe Tyr Ser Ser Leu Ile Ser
1025                1030                1035                1040

Tyr Glu Glu Asp Gln Arg Gln Gly Ala Glu Pro Arg Lys Asn Phe Val
                1045                1050                1055

Lys Pro Asn Glu Thr Lys Thr Tyr Phe Trp Lys Val Gln His His Met
            1060                1065                1070

Ala Pro Thr Lys Asp Glu Phe Asp Cys Lys Ala Trp Ala Tyr Phe Ser
        1075                1080                1085

Asp Val Asp Leu Glu Lys Asp Val His Ser Gly Leu Ile Gly Pro Leu
    1090                1095                1100

Leu Val Cys His Thr Asn Thr Leu Asn Pro Ala His Gly Arg Gln Val

1105                1110                1115                1120

Thr Val Gln Glu Phe Ala Leu Phe Phe Thr Ile Phe Asp Glu Thr Lys
                1125                1130                1135

Ser Trp Tyr Phe Thr Glu Asn Met Glu Arg Asn Cys Arg Ala Pro Cys
            1140                1145                1150

Asn Ile Gln Met Glu Asp Pro Thr Phe Lys Glu Asn Tyr Arg Phe His
        1155                1160                1165

Ala Ile Asn Gly Tyr Ile Met Asp Thr Leu Pro Gly Leu Val Met Ala
    1170                1175                1180

Gln Asp Gln Arg Ile Arg Trp Tyr Leu Leu Ser Met Gly Ser Asn Glu
1185                1190                1195                1200

Asn Ile His Ser Ile His Phe Ser Gly His Val Phe Thr Val Arg Lys
                1205                1210                1215

Lys Glu Glu Tyr Lys Met Ala Leu Tyr Asn Leu Tyr Pro Gly Val Phe
            1220                1225                1230

Glu Thr Val Glu Met Leu Pro Ser Lys Ala Gly Ile Trp Arg Val Glu
        1235                1240                1245

Cys Leu Ile Gly Glu His Leu His Ala Gly Met Ser Thr Leu Phe Leu
    1250                1255                1260

Val Tyr Ser Asn Lys Cys Gln Thr Pro Leu Gly Met Ala Ser Gly His
1265                1270                1275                1280

Ile Arg Asp Phe Gln Ile Thr Ala Ser Gly Gln Tyr Gly Gln Trp Ala
                1285                1290                1295

Pro Lys Leu Ala Arg Leu His Tyr Ser Gly Ser Ile Asn Ala Trp Ser
            1300                1305                1310

Thr Lys Glu Pro Phe Ser Trp Ile Lys Val Asp Leu Leu Ala Pro Met
        1315                1320                1325

Ile Ile His Gly Ile Lys Thr Gln Gly Ala Arg Gln Lys Phe Ser Ser
    1330                1335                1340

Leu Tyr Ile Ser Gln Phe Ile Ile Met Tyr Ser Leu Asp Gly Lys Lys
1345                1350                1355                1360

Trp Gln Thr Tyr Arg Gly Asn Ser Thr Gly Thr Leu Met Val Phe Phe
                1365                1370                1375

Gly Asn Val Asp Ser Ser Gly Ile Lys His Asn Ile Phe Asn Pro Pro
            1380                1385                1390

Ile Ile Ala Arg Tyr Ile Arg Leu His Pro Thr His Tyr Ser Ile Arg
        1395                1400                1405

Ser Thr Leu Arg Met Glu Leu Met Gly Cys Asp Leu Asn Ser Cys Ser
    1410                1415                1420

Met Pro Leu Gly Met Glu Ser Lys Ala Ile Ser Asp Ala Gln Ile Thr
1425                1430                1435                1440

Ala Ser Ser Tyr Phe Thr Asn Met Phe Ala Thr Trp Ser Pro Ser Lys

1445                    1450                    1455

Ala Arg Leu His Leu Gln Gly Arg Ser Asn Ala Trp Arg Pro Gln Val
        1460                    1465                    1470

Asn Asn Pro Lys Glu Trp Leu Gln Val Asp Phe Gln Lys Thr Met Lys
    1475                    1480                    1485

Val Thr Gly Val Thr Thr Gln Gly Val Lys Ser Leu Leu Thr Ser Met
1490                    1495                    1500

Tyr Val Lys Glu Phe Leu Ile Ser Ser Ser Gln Asp Gly His Gln Trp
1505                    1510                    1515                    1520

Thr Leu Phe Phe Gln Asn Gly Lys Val Lys Val Phe Gln Gly Asn Gln
                1525                    1530                    1535

Asp Ser Phe Thr Pro Val Val Asn Ser Leu Asp Pro Pro Leu Leu Thr
        1540                    1545                    1550

Arg Tyr Leu Arg Ile His Pro Gln Ser Trp Val His Gln Ile Ala Leu
    1555                    1560                    1565

Arg Met Glu Val Leu Gly Cys Glu Ala Gln Asp Leu Tyr
1570                    1575                    1580

<210>    9
<211>    4404
<212>    DNA
<213>    Artificial Sequence

<220>
<223>    G2

<400>    9
gccaccagaa gatactacct gggtgcagtg gaactgtcat gggactatat gcaaagtgat      60

ctcggtgagc tgcctgtgga cgcaagattt cctcctagag tgccaaaatc ttttccattc     120

aacacctcag tcgtgtacaa aaagactctg tttgtagaat tcacggatca ccttttcaac     180

atcgctaagc caaggccacc ctggatgggt ctgctaggtc ctaccatcca ggctgaggtt     240

tatgatacag tggtcattac acttaagaac atggcttccc atcctgtcag tcttcatgct     300

gttggtgtat cctactggaa agcttctgag ggagctgaat atgatgatca gaccagtcaa     360

agggagaaag aagatgataa agtcttccct ggtggaagcc atacatatgt ctggcaggtc     420

ctgaaagaga atggtccaat ggcctctgac ccactgtgcc ttacctactc atatctttct     480

catgtggacc tggtaaaaga cttgaattca ggcctcattg gagccctact agtatgtaga     540

gaagggagtc tggccaagga aaagacacag accttgcaca aatttatact actttttgct     600

gtatttgatg aagggaaaag ttggcactca gaaacaaaga actccttgat gcaggatagg     660

gatgctgcat ctgctcgggc ctggcctaaa atgcacacag tcaatggtta tgtaaacagg     720

tctctgccag gtctgattgg atgccacagg aaatcagtct attggcatgt gattggaatg     780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtactctt cagtcagatc aagaggaaat tgactatgat 2400

gataccatat cagttgaaat gaagaaggaa gattttgaca tttatgatga ggatgaaaat 2460

cagagccccc gcagctttca aaagaaaaca cgacactatt ttattgctgc agtggagagg 2520

ctctgggatt atgggatgag tagctcccca catgttctaa gaaacagggc tcagagtggc 2580

agtgtccctc agttcaagaa agttgttttc caggaattta ctgatggctc ctttactcag 2640

cccttatacc gtggagaact aaatgaacat ttgggactcc tggggccata tataagagca 2700

gaagttgaag ataatatcat ggtaactttc agaaatcagg cctctcgtcc ctattccttc 2760

tattctagcc ttatttctta tgaggaagat cagaggcaag gagcagaacc tagaaaaaac 2820

tttgtcaagc ctaatgaaac caaaacttac ttttggaaag tgcaacatca tatggcaccc 2880

actaaagatg agtttgactg caaagcctgg gcttatttct ctgatgttga cctggaaaaa 2940

gatgtgcact caggcctgat tggacccctt ctggtctgcc acactaacac actgaaccct 3000

gctcatggga gacaagtgac agtacaggaa tttgctctgt ttttcaccat ctttgatgag 3060

accaaaagct ggtacttcac tgaaaatatg gaaagaaact gcagggctcc ctgcaatatc 3120

cagatggaag atcccacttt taaagagaat tatcgcttcc atgcaatcaa tggctacata 3180

atggatacac tacctggctt agtaatggct caggatcaaa ggattcgatg gtatctgctc 3240

agcatgggca gcaatgaaaa catccattct attcatttca gtggacatgt gttcactgta 3300

cgaaaaaaag aggagtataa aatggcactg tacaatctct atccaggtgt ttttgagaca 3360

gtggaaatgt taccatccaa agctggaatt tggcgggtgg aatgccttat tggcgagcat 3420

ctacatgctg ggatgagcac actttttctg gtgtacagca ataagtgtca gactcccctg 3480

ggaatggctt ctggacacat tagagatttt cagattacag cttcaggaca atatggacag 3540

tgggccccaa agctggccag acttcattat tccggatcaa tcaatgcctg gagcaccaag 3600

gagccctttt cttggatcaa ggtggatctg ttggcaccaa tgattattca cggcatcaag 3660

acccagggtg cccgtcagaa gttctccagc ctctacatct ctcagtttat catcatgtat 3720

agtcttgatg ggaagaagtg gcagacttat cgaggaaatt ccactggaac cttaatggtc 3780

ttctttggca atgtggattc atctgggata aaacacaata tttttaaccc tccaattatt 3840

gctcgataca tccgtttgca cccaactcat tatagcattc gcagcactct tcgcatggag 3900

ttgatgggct gtgatttaaa tagttgcagc atgccattgg gaatggagag taaagcaata 3960

tcagatgcac agattactgc ttcatcctac tttaccaata tgtttgccac ctggtctcct 4020

tcaaaagctc gacttcacct ccaagggagg agtaatgcct ggagacctca ggtgaataat 4080

ccaaaagagt ggctgcaagt ggacttccag aagacaatga aagtcacagg agtaactact 4140

cagggagtaa aatctctgct taccagcatg tatgtgaagg agttcctcat ctccagcagt 4200

caagatggcc atcagtggac tctctttttt cagaatggca aagtaaaggt tttcagggga 4260

aatcaagact ccttcacacc tgtggtgaac tctctagacc caccgttact gactcgctac 4320

cttcgaattc acccccagag ttgggtgcac cagattgccc tgaggatgga ggttctgggc 4380

tgcgaggcac aggacctcta ctga 4404

<210> 10
<211> 4536

<212>     DNA
<213>     Artificial Sequence

<220>
<223>     G3


<400>     10
gccaccagaa gatactacct gggtgcagtg gaactgtcat gggactatat gcaaagtgat          60

ctcggtgagc tgcctgtgga cgcaagattt cctcctagag tgccaaaatc ttttccattc         120

aacacctcag tcgtgtacaa aaagactctg tttgtagaat tcacggatca ccttttcaac         180

atcgctaagc caaggccacc ctggatgggt ctgctaggtc ctaccatcca ggctgaggtt         240

tatgatacag tggtcattac acttaagaac atggcttccc atcctgtcag tcttcatgct         300

gttggtgtat cctactggaa agcttctgag ggagctgaat atgatgatca gaccagtcaa         360

agggagaaag aagatgataa agtcttccct ggtggaagcc atacatatgt ctggcaggtc         420

ctgaaagaga atggtccaat ggcctctgac ccactgtgcc ttacctactc atatctttct         480

catgtggacc tggtaaaaga cttgaattca ggcctcattg gagccctact agtatgtaga         540

gaagggagtc tggccaagga aaagacacag accttgcaca aatttatact actttttgct          600

gtatttgatg aagggaaaag ttggcactca gaaacaaaga actccttgat gcaggatagg         660

gatgctgcat ctgctcgggc ctggcctaaa atgcacacag tcaatggtta tgtaaacagg         720

tctctgccag gtctgattgg atgccacagg aaatcagtct attggcatgt gattggaatg         780

ggcaccactc ctgaagtgca ctcaatattc ctcgaaggtc acacatttct tgtgaggaac         840

catcgccagg cgtccttgga aatctcgcca ataactttcc ttactgctca aacactcttg         900

atggaccttg gacagtttct actgttttgt catatctctt cccaccaaca tgatggcatg         960

gaagcttatg tcaaagtaga cagctgtcca gaggaacccc aactacgaat gaaaaataat        1020

gaagaagcgg aagactatga tgatgatctt actgattctg aaatggatgt ggtcaggttt        1080

gatgatgaca actctccttc ctttatccaa attcgctcag ttgccaagaa gcatcctaaa        1140

acttgggtac attacattgc tgctgaagag gaggactggg actatgctcc cttagtcctc        1200

gcccccgatg acagaagtta taaaagtcaa tatttgaaca atggccctca gcggattggt        1260

aggaagtaca aaaaagtccg atttatggca tacacagatg aaacctttaa gactcgtgaa        1320

gctattcagc atgaatcagg aatcttggga cctttacttt atggggaagt tggagacaca        1380

ctgttgatta tatttaagaa tcaagcaagc agaccatata acatctaccc tcacggaatc        1440

actgatgtcc gtcctttgta ttcaaggaga ttaccaaaag gtgtaaaaca tttgaaggat        1500

tttccaattc tgccaggaga aatattcaaa tataaatgga cagtgactgt agaagatggg        1560

ccaactaaat cagatcctcg gtgcctgacc cgctattact ctagtttcgt taatatggag        1620

agagatctag cttcaggact cattggccct ctcctcatct gctacaaaga atctgtagat        1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctactc ttcagtcaga tcaagaggaa 2520

attgactatg atgataccat atcagttgaa atgaagaagg aagattttga catttatgat 2580

gaggatgaaa atcagagccc ccgcagcttt caaaagaaaa cacgacacta ttttattgct 2640

gcagtggaga ggctctggga ttatgggatg agtagctccc cacatgttct aagaaacagg 2700

gctcagagtg gcagtgtccc tcagttcaag aaagttgttt tccaggaatt tactgatggc 2760

tcctttactc agcccttata ccgtggagaa ctaaatgaac atttgggact cctggggcca 2820

tatataagag cagaagttga agataatatc atggtaactt tcagaaatca ggcctctcgt 2880

ccctattcct tctattctag ccttatttct tatgaggaag atcagaggca aggagcagaa 2940

cctagaaaaa actttgtcaa gcctaatgaa accaaaactt acttttggaa agtgcaacat 3000

catatggcac ccactaaaga tgagtttgac tgcaaagcct gggcttattt ctctgatgtt 3060

gacctggaaa aagatgtgca ctcaggcctg attggacccc ttctggtctg ccacactaac 3120

acactgaacc ctgctcatgg gagacaagtg acagtacagg aatttgctct gtttttcacc 3180

atctttgatg agaccaaaag ctggtacttc actgaaaata tggaaagaaa ctgcagggct 3240

ccctgcaata tccagatgga agatcccact tttaaagaga attatcgctt ccatgcaatc 3300

aatggctaca taatggatac actacctggc ttagtaatgg ctcaggatca aaggattcga 3360

tggtatctgc tcagcatggg cagcaatgaa aacatccatt ctattcattt cagtggacat 3420

gtgttcactg tacgaaaaaa agaggagtat aaaatggcac tgtacaatct ctatccaggt 3480

gtttttgaga cagtggaaat gttaccatcc aaagctggaa tttggcgggt ggaatgcctt 3540

attggcgagc atctacatgc tgggatgagc acactttttc tggtgtacag caataagtgt 3600

cagactcccc tgggaatggc ttctggacac attagagatt ttcagattac agcttcagga 3660

caatatggac agtgggcccc aaagctggcc agacttcatt attccggatc aatcaatgcc 3720

tggagcacca aggagccctt ttcttggatc aaggtggatc tgttggcacc aatgattatt 3780

cacggcatca agacccaggg tgcccgtcag aagttctcca gcctctacat ctctcagttt 3840

atcatcatgt atagtcttga tgggaagaag tggcagactt atcgaggaaa ttccactgga 3900

accttaatgg tcttctttgg caatgtggat tcatctggga taaaacacaa tatttttaac 3960

cctccaatta ttgctcgata catccgtttg cacccaactc attatagcat tcgcagcact 4020

cttcgcatgg agttgatggg ctgtgattta aatagttgca gcatgccatt gggaatggag 4080

agtaaagcaa tatcagatgc acagattact gcttcatcct actttaccaa tatgtttgcc 4140

acctggtctc cttcaaaagc tcgacttcac ctccaaggga ggagtaatgc ctggagacct 4200

caggtgaata atccaaaaga gtggctgcaa gtggacttcc agaagacaat gaaagtcaca 4260

ggagtaacta ctcagggagt aaaatctctg cttaccagca tgtatgtgaa ggagttcctc 4320

atctccagca gtcaagatgg ccatcagtgg actctctttt ttcagaatgg caaagtaaag 4380

gtttttcagg gaaatcaaga ctccttcaca cctgtggtga actctctaga cccaccgtta 4440

ctgactcgct accttcgaat tcacccccag agttgggtgc accagattgc cctgaggatg 4500

gaggttctgg gctgcgaggc acaggacctc tactga 4536

<210> 11
<211> 4746
<212> DNA
<213> Artificial Sequence

<220>
<223> G4

<400> 11
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

<210> 12
<211> 4935
<212> DNA
<213> Artificial Sequence

<220>
<223> G6

<400> 12
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagttcct taggaccccc aagtatgcca gttcattatg atagtcaatt agataccact 2760

ctatttggca aaaagtcatc tccccttact gagtctggtg gacctctgag cttgagtgaa 2820

gaaaataatg attcaaagtt gttagaatca ggtttaatga atagccaaga aagttcatgg 2880

ggaaaaaatg tatcgactct tcagtcagat caagaggaaa ttgactatga tgataccata 2940

tcagttgaaa tgaagaagga agattttgac atttatgatg aggatgaaaa tcagagcccc 3000

cgcagctttc aaaagaaaac acgacactat tttattgctg cagtggagag gctctgggat 3060

tatgggatga gtagctcccc acatgttcta agaaacaggg ctcagagtgg cagtgtccct 3120

cagttcaaga aagttgtttt ccaggaattt actgatggct cctttactca gcccttatac 3180

cgtggagaac taaatgaaca tttgggactc ctggggccat atataagagc agaagttgaa 3240

gataatatca tggtaacttt cagaaatcag gcctctcgtc cctattcctt ctattctagc 3300

cttatttctt atgaggaaga tcagaggcaa ggagcagaac ctagaaaaaa ctttgtcaag 3360

cctaatgaaa ccaaaactta cttttggaaa gtgcaacatc atatggcacc cactaaagat 3420

gagtttgact gcaaagcctg ggcttatttc tctgatgttg acctggaaaa agatgtgcac 3480

tcaggcctga ttggacccct tctggtctgc cacactaaca cactgaaccc tgctcatggg 3540

agacaagtga cagtacagga atttgctctg tttttcacca tctttgatga gaccaaaagc 3600

tggtacttca ctgaaaatat ggaaagaaac tgcagggctc cctgcaatat ccagatggaa 3660

gatcccactt ttaaagagaa ttatcgcttc catgcaatca atggctacat aatggataca 3720

ctacctggct tagtaatggc tcaggatcaa aggattcgat ggtatctgct cagcatgggc 3780

agcaatgaaa acatccattc tattcatttc agtggacatg tgttcactgt acgaaaaaaa 3840

gaggagtata aaatggcact gtacaatctc tatccaggtg tttttgagac agtggaaatg 3900

ttaccatcca aagctggaat ttggcgggtg gaatgcctta ttggcgagca tctacatgct 3960

gggatgagca cactttttct ggtgtacagc aataagtgtc agactcccct gggaatggct 4020

tctggacaca ttagagattt tcagattaca gcttcaggac aatatggaca gtgggcccca 4080

aagctggcca gacttcatta ttccggatca atcaatgcct ggagcaccaa ggagcccttt 4140

tcttggatca aggtggatct gttggcacca atgattattc acggcatcaa gacccagggt 4200

gcccgtcaga agttctccag cctctacatc tctcagttta tcatcatgta tagtcttgat 4260

gggaagaagt ggcagactta tcgaggaaat tccactggaa ccttaatggt cttctttggc 4320

aatgtggatt catctgggat aaaacacaat atttttaacc ctccaattat tgctcgatac 4380

atccgtttgc acccaactca ttatagcatt cgcagcactc ttcgcatgga gttgatgggc 4440

tgtgatttaa atagttgcag catgccattg ggaatggaga gtaaagcaat atcagatgca 4500

cagattactg cttcatccta ctttaccaat atgtttgcca cctggtctcc ttcaaaagct 4560

cgacttcacc tccaagggag gagtaatgcc tggagacctc aggtgaataa tccaaaagag 4620

tggctgcaag tggacttcca gaagacaatg aaagtcacag gagtaactac tcagggagta 4680

aaatctctgc ttaccagcat gtatgtgaag gagttcctca tctccagcag tcaagatggc 4740

catcagtgga ctctcttttt tcagaatggc aaagtaaagg tttttcaggg aaatcaagac 4800

tccttcacac ctgtggtgaa ctctctagac ccaccgttac tgactcgcta ccttcgaatt 4860

cacccccaga gttgggtgca ccagattgcc ctgaggatgg aggttctggg ctgcgaggca 4920

caggacctct actga 4935

<210> 13
<211> 4764
<212> DNA
<213> Artificial Sequence

<220>
<223> G4-flex

<400> 13
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaaatacaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagttctc aaaacccacc agtcactctt cagtcagatc aagaggaaat tgactatgat 2760

gataccatat cagttgaaat gaagaaggaa gattttgaca tttatgatga ggatgaaaat 2820

cagagccccc gcagctttca aaagaaaaca cgacactatt ttattgctgc agtggagagg 2880

ctctgggatt atgggatgag tagctcccca catgttctaa gaaacagggc tcagagtggc 2940

agtgtccctc agttcaagaa agttgttttc caggaattta ctgatggctc ctttactcag 3000

cccttatacc gtggagaact aaatgaacat ttgggactcc tggggccata tataagagca 3060

gaagttgaag ataatatcat ggtaactttc agaaatcagg cctctcgtcc ctattccttc 3120

tattctagcc ttatttctta tgaggaagat cagaggcaag gagcagaacc tagaaaaaac 3180

tttgtcaagc ctaatgaaac caaaacttac ttttggaaag tgcaacatca tatggcaccc 3240

actaaagatg agtttgactg caaagcctgg gcttatttct ctgatgttga cctggaaaaa 3300

gatgtgcact caggcctgat tggacccctt ctggtctgcc acactaacac actgaaccct 3360

gctcatggga gacaagtgac agtacaggaa tttgctctgt ttttcaccat ctttgatgag 3420

accaaaagct ggtacttcac tgaaaatatg gaaagaaact gcagggctcc ctgcaatatc 3480

cagatggaag atcccacttt taaagagaat tatcgcttcc atgcaatcaa tggctacata 3540

atggatacac tacctggctt agtaatggct caggatcaaa ggattcgatg gtatctgctc 3600

agcatgggca gcaatgaaaa catccattct attcatttca gtggacatgt gttcactgta 3660

cgaaaaaaag aggagtataa aatggcactg tacaatctct atccaggtgt ttttgagaca 3720

gtggaaatgt taccatccaa agctggaatt tggcgggtgg aatgccttat tggcgagcat 3780

ctacatgctg ggatgagcac actttttctg gtgtacagca ataagtgtca gactcccctg 3840

ggaatggctt ctggacacat tagagatttt cagattacag cttcaggaca atatggacag 3900

tgggccccaa agctggccag acttcattat tccggatcaa tcaatgcctg gagcaccaag 3960

gagccctttt cttggatcaa ggtggatctg ttggcaccaa tgattattca cggcatcaag 4020

acccagggtg cccgtcagaa gttctccagc ctctacatct ctcagtttat catcatgtat 4080

agtcttgatg ggaagaagtg gcagacttat cgaggaaatt ccactggaac cttaatggtc 4140

ttctttggca atgtggattc atctgggata aaacacaata tttttaaccc tccaattatt 4200

gctcgataca tccgtttgca cccaactcat tatagcattc gcagcactct tcgcatggag 4260

ttgatgggct gtgatttaaa tagttgcagc atgccattgg gaatggagag taaagcaata 4320

tcagatgcac agattactgc ttcatcctac tttaccaata tgtttgccac ctggtctcct 4380

tcaaaagctc gacttcacct ccaagggagg agtaatgcct ggagacctca ggtgaataat 4440

ccaaaagagt ggctgcaagt ggacttccag aagacaatga aagtcacagg agtaactact 4500

cagggagtaa aatctctgct taccagcatg tatgtgaagg agttcctcat ctccagcagt 4560

caagatggcc atcagtggac tctctttttt cagaatggca aagtaaaggt ttttcaggga 4620

aatcaagact ccttcacacc tgtggtgaac tctctagacc caccgttact gactcgctac 4680

cttcgaattc acccccagag ttgggtgcac cagattgccc tgaggatgga ggttctgggc 4740

tgcgaggcac aggacctcta ctga 4764

<210> 14
<211> 4935
<212> DNA
<213> Artificial Sequence

<220>
<223> G6_opt

<400> 14
gccacccggc ggtactacct gggcgccgtg gaactgtcct gggactacat gcagtccgac 60

ctgggcgagc tgcccgtgga cgccagattc cccccaagag tgcccaagtc cttccccttc 120

aacacctccg tggtgtacaa gaaaaccctg ttcgtggaat tcaccgacca cctgttcaat 180

atcgccaagc ctcggccccc ttggatgggc ctgctgggcc ctacaatcca ggccgaggtg 240

tacgacaccg tggtgatcac cctgaagaac atggcctccc accccgtgtc cctgcacgcc 300

gtgggcgtgt cctactggaa ggcctctgag ggcgccgagt acgacgacca gacctcccag 360

cgcgagaaag aggacgacaa ggtgttccct ggcggctccc acacctacgt gtggcaggtg 420

ctgaaagaaa acggccctat ggcctccgac cctctgtgcc tgacctactc ctacctgtcc 480

cacgtggacc tggtgaaaga cctgaactcc ggcctgatcg gcgccctgct ggtgtgcaga 540

gagggctccc tggccaaaga aaagacccag accctgcaca agttcatcct gctgttcgcc 600

gtgttcgacg agggcaagtc ctggcactcc gagacaaaga actccctgat gcaggaccgg 660

gacgccgcct ctgccagagc ctggcctaag atgcacaccg tgaacggcta cgtgaaccgg 720

tccctgcccg gactgatcgg ctgccaccgg aagtccgtgt actggcacgt gatcggcatg 780

ggcaccaccc ccgaggtgca ctccatcttt ctggaaggcc acacctttct ggtgcgaaac 840

caccggcagg cctccctgga aatctcccct atcaccttcc tgaccgctca gaccctgctg 900

atggacctgg gccagttcct gctgttttgc cacatctcca gccaccagca cgacggcatg 960

gaagcctacg tgaaggtgga ctcctgcccc gaggaacccc agctgcggat gaagaacaac 1020

gaggaagccg aggactacga cgacgacctg accgactccg agatggacgt ggtgcgattc 1080

gacgacgaca actcccccag cttcatccag atcagatccg tggccaagaa gcaccccaag 1140

acctgggtgc actatatcgc cgccgaggaa gaggactggg actacgcccc tctggtgctg 1200

gcccctgacg accggtccta caagtcccag tacctgaaca acggccccca gcggatcggc 1260

cggaagtaca agaaagtgcg gttcatggcc tacaccgacg agacattcaa gaccagagag 1320

gccatccagc acgagtccgg catcctgggc cccctgctgt acggcgaagt gggcgacaca 1380

ctgctgatca tcttcaagaa ccaggcctcc cggccctaca acatctaccc ccacggcatc 1440

accgacgtgc ggcccctgta ctctcggcgg ctgcctaagg gcgtgaagca cctgaaggac 1500

ttccccatcc tgcccggcga gatcttcaag tacaagtgga ccgtgaccgt ggaagatggc 1560

cccaccaagt ccgaccccag atgcctgacc cggtactact cctccttcgt gaacatggaa 1620

cgggacctgg ccagcggcct gattggacct ctgctgatct gctacaaaga atccgtggac 1680

cagcggggca accagatcat gtccgacaag cggaacgtga tcctgttcag cgtgttcgat 1740

gagaaccggt cctggtatct gaccgagaac atccagcggt ttctgcccaa ccctgccggc 1800

gtgcagctgg aagatcccga gttccaggcc tccaacatca tgcacagcat caatggctac 1860

gtgttcgact ccctgcagct gagcgtgtgc ctgcacgagg tggcctactg gtacatcctg 1920

tccatcggcg cccagaccga cttcctgtcc gtgttcttct ccggctacac cttcaagcac 1980

aagatggtgt acgaggatac cctgaccctg ttccccttct ccggcgaaac cgtgttcatg 2040

tccatggaaa accccggcct gtggatcctg ggctgccaca actccgactt ccggaaccgg 2100

ggcatgaccg ccctgctgaa ggtgtcctcc tgcgacaaga acaccggcga ctactacgag 2160

gactcttacg aggacatctc cgcctacctg ctgtccaaga acaacgccat cgagccccgg 2220

tccttcagcc agaactcccg gcacccttcc acccggcaga agcagttcaa cgccaccacc 2280

atccccgaga acgacatcga aaagaccgac ccttggtttg cccaccggac ccccatgccc 2340

aagatccaga acgtgtcctc ctctgacctg ctgatgctgc tgcggcagtc ccccacccct 2400

cacggcctgt ctctgagcga cctgcaggaa gctaagtacg agactttctc cgacgacccc 2460

tcccctggcg ccatcgactc caacaactcc ctgtccgaga tgacccactt ccggccccag 2520

ctgcaccact ccggcgacat ggtgttcacc cccgagtctg gcctgcagct gcggctgaac 2580

gagaagctgg gcaccacagc cgccaccgag ctgaagaaac tggacttcaa ggtgtccagc 2640

acctccaaca acctgatctc caccatccct tccgacaacc tggccgctgg caccgacaac 2700

acctcttccc tgggccctcc ctccatgccc gtgcactacg actcccagct ggacaccacc 2760

ctgttcggca agaagtccag ccccctgacc gagtccggcg gacccctgtc cctgtctgag 2820

gaaaacaacg actccaagct gctggaatct ggcctgatga actcccagga atcctcctgg 2880

ggcaagaatg tgtccaccct gcagagcgac caggaagaga tcgattacga cgacaccatc 2940

tccgtggaaa tgaagaaaga ggatttcgac atctacgacg aggacgagaa ccagtcccct 3000

cggtccttcc agaagaaaac ccggcactac tttatcgccg ctgtggaacg gctgtgggac 3060

tacggcatgt cctcctcccc ccacgtgctg cggaacagag cccagtccgg ctccgtgccc 3120

cagttcaaga aagtggtgtt ccaggaattc acagacggca gcttcaccca gcccctgtac 3180

cggggcgagc tgaatgagca cctgggactg ctgggacctt acatcagagc tgaggtggaa 3240

gataacatca tggtgacatt ccggaaccag gccagcagac cctactcctt ctactccagc 3300

ctgatctcct acgaagagga ccagcggcag ggcgctgagc cccggaagaa cttcgtgaag 3360

cccaacgaga ctaagaccta cttctggaag gtgcagcacc acatggctcc taccaaggac 3420

gagttcgact gcaaggcctg ggcctacttc tccgatgtgg acctggaaaa ggacgtgcac 3480

agcgggctga ttggcccact gctggtgtgc cacaccaaca ccctgaaccc cgcccacggc 3540

agacaggtga cagtgcagga attcgccctg ttcttcacca tcttcgacga gacaaagtcc 3600

tggtacttca ccgagaatat ggaacggaac tgcagagccc cctgcaacat ccagatggaa 3660

gatcctacct tcaaagagaa ctaccggttc cacgccatca acggctacat catggacacc 3720

ctgcccggcc tggtgatggc ccaggaccag agaatccggt ggtatctgct gtccatgggc 3780

tccaacgaga atatccactc catccacttc tccggccacg tgttcaccgt gcggaagaaa 3840

gaagagtaca agatggccct gtacaacctg taccccggcg tgttcgagac agtggaaatg 3900

ctgccctcca aggccggcat ctggcgggtg gaatgtctga tcggcgagca tctgcacgcc 3960

ggaatgtcta ccctgttcct ggtgtactcc aacaagtgcc agacccccct gggcatggcc 4020

tccggccaca tccgggattt ccagatcacc gcctctggcc agtacggcca gtgggcccct 4080

aagctggccc ggctgcacta cagcggctcc atcaacgcct ggtctaccaa agagcccttc 4140

agctggatca aggtggacct gctggccccc atgatcatcc atggcatcaa gacccagggc 4200

gccagacaga agttctcctc cctgtacatc tcccagttca tcatcatgta ctccctggat 4260

ggcaagaagt ggcagaccta ccggggcaac tccaccggca ccctgatggt gttcttcggc 4320

aacgtggact cctccggcat caagcacaac atcttcaacc cccccatcat tgcccggtac 4380

atccggctgc accccaccca ctactccatc cggtctaccc tgcggatgga actgatgggc 4440

tgcgacctga acagctgctc catgcctctg ggcatggaat ccaaggccat ctccgacgcc 4500

cagatcacag ccagctccta cttcaccaac atgttcgcca cctggtcccc cagcaaggcc 4560

agactgcatc tgcagggccg gtccaacgct tggcggcctc aggtgaacaa ccccaaagaa 4620

tggctgcagg tggacttcca gaaaaccatg aaggtgacag gcgtgaccac ccagggcgtg 4680

aaatccctgc tgacctctat gtacgtgaaa gagttcctga tcagctcctc ccaggacggc 4740

caccagtgga ccctgttctt tcagaacggc aaagtgaaag tgttccaggg caaccaggac 4800

tccttcaccc ctgtggtgaa ctccctggac cccccactgc tgaccagata cctgcggatc 4860

cacccccagt cttgggtgca ccagatcgcc ctgagaatgg aagtgctggg atgcgaggcc 4920

caggatctgt actga 4935

<210> 15
<211> 4746
<212> DNA
<213> Artificial Sequence

<220>
<223> G4_B3

<400> 15
gccaccagaa gatactacct gggtgcagtg gaactgtcat gggactatat gcaaagtgat 60

ctcggtgagc tgcctgtgga cgcaagattt cctcctagag tgccaaaatc ttttccattc 120

aacacctcag tcgtgtacaa aaagactctg tttgtagaat tcacggatca ccttttcaac 180

atcgctaagc caaggccacc ctggatgggt ctgctaggtc ctaccatcca ggctgaggtt 240

tatgatacag tggtcattac acttaagaac atggcttccc atcctgtcag tcttcatgct 300

gttggtgtat cctactggaa agcttctgag ggagctgaat atgatgatca gaccagtcaa 360

agggagaaag aagatgataa agtcttccct ggtggaagcc atacatatgt ctggcaggtc 420

ctgaaagaga atggtccaat ggcctctgac ccactgtgcc ttacctactc atatctttct 480

catgtggacc tggtaaaaga cttgaattca ggcctcattg gagccctact agtatgtaga 540

gaagggagtc tggccaagga aaagacacag accttgcaca aatttatact actttttgct 600

gtatttgatg aagggaaaag ttggcactca gaaacaaaga actccttgat gcaggatagg 660

gatgctgcat ctgctcgggc ctggcctaaa atgcacacag tcaatggtta tgtaaacagg 720

tctctgccag gtctgattgg atgccacagg aaatcagtct attggcatgt gattggaatg 780

ggcaccactc ctgaagtgca ctcaatattc ctcgaaggtc acacatttct tgtgaggaac 840

catcgccagg cgtccttgga aatctcgcca ataactttcc ttactgctca aacactcttg 900

atggaccttg gacagtttct actgttttgt catatctctt cccaccaaca tgatggcatg 960

gaagcttatg tcaaagtaga cagctgtcca gaggaacccc aactacgaat gaaaaataat 1020

gaagaagcgg aagactatga tgatgatctt actgattctg aaatggatgt ggtcaggttt 1080

gatgatgaca actctccttc ctttatccaa attcgctcag ttgccaagaa gcatcctaaa 1140

acttgggtac attacattgc tgctgaagag gaggactggg actatgctcc cttagtcctc 1200

gcccccgatg acagaagtta taaaagtcaa tatttgaaca atggccctca gcggattggt 1260

aggaagtaca aaaaagtccg atttatggca tacacagatg aaacctttaa gactcgtgaa 1320

gctattcagc atgaatcagg aatcttggga cctttacttt atggggaagt tggagacaca 1380

ctgttgatta tatttaagaa tcaagcaagc agaccatata acatctaccc tcacggaatc 1440

actgatgtcc gtcctttgta ttcaaggaga ttaccaaaag gtgtaaaaca tttgaaggat 1500

tttccaattc tgccaggaga aatattcaaa tataaatgga cagtgactgt agaagatggg 1560

ccaactaaat cagatcctcg gtgcctgacc cgctattact ctagtttcgt taatatggag 1620

agagatctag cttcaggact cattggccct ctcctcatct gctacaaaga atctgtagat 1680

caaagaggaa accagataat gtcagacaag aggaatgtca tcctgttttc tgtatttgat 1740

gagaaccgaa gctggtacct cacagagaat atacaacgct ttctccccaa tccagctgga 1800

gtgcagcttg aggatccaga gttccaagcc tccaacatca tgcacagcat caatggctat 1860

gtttttgata gtttgcagtt gtcagtttgt ttgcatgagg tggcatactg gtacattcta 1920

agcattggag cacagactga cttcctttct gtcttcttct ctggatatac cttcaaacac 1980

aaaatggtct atgaagacac actcacccta ttcccattct caggagaaac tgtcttcatg 2040

tcgatggaaa acccaggtct atggattctg gggtgccaca actcagactt tcggaacaga 2100

ggcatgaccg ccttactgaa ggtttctagt tgtgacaaga acactggtga ttattacgag 2160

gacagttatg aagatatttc agcatacttg ctgagtaaaa acaatgccat tgaaccaaga 2220

agcttctccc agaattcaag acaccctagc actaggcaaa agcaatttaa tgccaccaca 2280

attccagaaa atgacataga gaagactgac ccttggtttg cacacagaac acctatgcct 2340

aaatgtcaaa atgtctcctc tagtgatttg ttgatgctct tgcgacagag tcctactcca 2400

catgggctat ccttatctga tctccaagaa gccaaatatg agactttttc tgatgatcca 2460

tcacctggag caatagacag taataacagc ctgtctgaaa tgacacactt caggccacag 2520

ctccatcaca gtggggacat ggtatttacc cctgagtcag gcctccaatt aagattaaat 2580

gagaaactgg ggacaactgc agcaacagag ttgaagaaac ttgatttcaa agtttctagt 2640

acatcaaata atctgatttc aacaattcca tcagacaatt tggcagcagg tactgataat 2700

acaagtactc ttcagtcaga tcaagaggaa attgactatg atgataccat atcagttgaa 2760

atgaagaagg aagattttga catttatgat gaggatgaaa atcagagccc ccgcagcttt 2820

caaaagaaaa cacgacacta ttttattgct gcagtggaga ggctctggga ttatgggatg 2880

agtagctccc cacatgttct aagaaacagg gctcagagtg gcagtgtccc tcagttcaag 2940

aaagttgttt tccaggaatt tactgatggc tcctttactc agcccttata ccgtggagaa 3000

ctaaatgaac atttgggact cctggggcca tatataagag cagaagttga agataatatc 3060

atggtaactt tcagaaatca ggcctctcgt ccctattcct tctattctag ccttatttct 3120

tatgaggaag atcagaggca aggagcagaa cctagaaaaa actttgtcaa gcctaatgaa 3180

accaaaactt acttttggaa agtgcaacat catatggcac ccactaaaga tgagtttgac 3240

tgcaaagcct gggcttattt ctctgatgtt gacctggaaa aagatgtgca ctcaggcctg 3300

attggacccc ttctggtctg ccacactaac acactgaacc ctgctcatgg gagacaagtg 3360

acagtacagg aatttgctct gtttttcacc atctttgatg agaccaaaag ctggtacttc 3420

actgaaaata tggaaagaaa ctgcagggct ccctgcaata tccagatgga agatcccact 3480

tttaaagaga attatcgctt ccatgcaatc aatggctaca taatggatac actacctggc 3540

ttagtaatgg ctcaggatca aaggattcga tggtatctgc tcagcatggg cagcaatgaa 3600

aacatccatt ctattcattt cagtggacat gtgttcactg tacgaaaaaa agaggagtat 3660

aaaatggcac tgtacaatct ctatccaggt gtttttgaga cagtggaaat gttaccatcc 3720

aaagctggaa tttggcgggt ggaatgcctt attggcgagc atctacatgc tgggatgagc 3780

acactttttc tggtgtacag caataagtgt cagactcccc tgggaatggc ttctggacac 3840

attagagatt ttcagattac agcttcagga caatatggac agtgggcccc aaagctggcc 3900

agacttcatt attccggatc aatcaatgcc tggagcacca aggagccctt ttcttggatc 3960

aaggtggatc tgttggcacc aatgattatt cacggcatca agacccaggg tgcccgtcag 4020

aagttctcca gcctctacat ctctcagttt atcatcatgt atagtcttga tgggaagaag 4080

tggcagactt atcgaggaaa ttccactgga accttaatgg tcttctttgg caatgtggat 4140

tcatctggga taaaacacaa tatttttaac cctccaatta ttgctcgata catccgtttg 4200

cacccaactc attatagcat tcgcagcact cttcgcatgg agttgatggg ctgtgattta 4260

aatagttgca gcatgccatt gggaatggag agtaaagcaa tatcagatgc acagattact 4320

gcttcatcct actttaccaa tatgtttgcc acctggtctc cttcaaaagc tcgacttcac 4380

ctccaaggga ggagtaatgc ctggagacct caggtgaata atccaaaaga gtggctgcaa 4440

gtggacttcc agaagacaat gaaagtcaca ggagtaacta ctcagggagt aaaatctctg 4500

cttaccagca tgtatgtgaa ggagttcctc atctccagca gtcaagatgg ccatcagtgg 4560

actctctttt ttcagaatgg caaagtaaag gtttttcagg gaaatcaaga ctccttcaca 4620

cctgtggtga actctctaga cccaccgtta ctgactcgct accttcgaat tcacccccag 4680

agttgggtgc accagattgc cctgaggatg gaggttctgg gctgcgaggc acaggacctc 4740

tactga 4746

## Description

### Technical Field

The present invention provides an *in vivo* long-acting recombinant coagulation factor VIII having a structure in which a biocompatible polymer is connected to the terminus of at least one sugar chain, and a preparation method therefor.

### Background Art

A glycoprotein refers to a protein that contains a sugar chain on the surface, the sugar chain having been connected to a protein residue in the Golgi apparatus during the protein production process. In glycoproteins, the function of a sugar chain has a significant effect on protein properties. For example, a sugar chain may improve water solubility of proteins, protect proteins from proteases, decrease immunogenicity of proteins, determine directionality of intracellular transport and localization of proteins, or affect intercellular interactions (Function and 3D Structure of the N-Glycans on Glycoproteins, Int. J. Mol. Sci. 2012, 13, 8398-8429).

Due to this importance of sugar chain, attempts have been made to improve protein properties through glycoengineering. For example, for erythropoietin (EPO), introduction of two additional N-sugar chains thereinto can improve its half-life in the body (Glycoengineering: the effect of glycosylation on the properties of therapeutic proteins, J Pharm Sci. 2005 94(8): 1626-35). In addition, it has been reported that in case of cancer cell-targeted antibody therapeutics, antibodies lacking fucose, which have been produced through glycoengineering, better induce the host's immune response and have a better therapeutic effect (Production of therapeutic antibodies with controlled fucosylation, MAbs. 2009 1(3): 230-236). These methods using glycoengineering have advantages in that generation of byproducts caused by side reactions can be minimized and such methods are efficient, as compared with conventional chemical methods.

Meanwhile, regarding methods for improving glycoprotein properties through changes in sugar chain, attempts have been made to apply metabolic glycoengineering so that changes in sugar chain are implemented in a more sophisticated way; however, specific applications and commercial uses thereof have not yet been sufficient.

### Disclosure of Invention

### Technical Problem

An object of the present invention is to provide an *in vivo* long-acting recombinant glycoprotein in which a biocompatible polymer is connected to a sialic acid derivative introduced to the terminus of at least one sugar chain, preferably a coagulation factor VIII in which a biocompatible polymer is connected to a sialic acid derivative introduced to the terminus of at least one sugar chain.

Another object of the present invention is to provide a method for preparing an *in vivo* long-acting recombinant coagulation factor VIII, in which a metabolic labeling technique is applied so that sialic acid at the terminus of at least one sugar chain of a coagulation factor VIII is modified to a sialic acid derivative to which an azide or alkyne group is introduced.

Yet another object of the present invention is to provide a method for preparing a modified *in vivo* long-acting recombinant coagulation factor VIII, in which the modification is done by introducing a biocompatible polymer to the sialic acid derivative at the terminus of sugar chain.

### Solution to Problem

In order to achieve the above-mentioned object, in an embodiment of the present invention, there is provided an *in vivo* long-acting recombinant coagulation factor VIII, comprising a linker which is linked to a sialic acid derivative at the terminus of at least one sugar chain of a coagulation factor VIII, wherein the linker is represented by Formula 1: 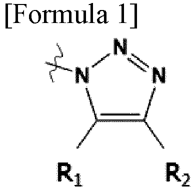 where R₁ and R₂ are each independently C₁₋₈ alkyl, C₆₋₁₂ aryl, C₃₋₆ cycloalkyl, 3- to 10-membered heterocycloalkyl, amino, (amino)C₁₋₆ alkyl, (amino)C₃₋₆ cycloalkyl, (amino)C₆₋₁₂ aryl, (amino)(3- to 10-membered heterocycloalkyl), or 5-to 12-membered heteroaryl (in which the heterocycloalkyl and the heteroaryl may each independently contain at least one heteroatom selected from the group consisting of N, S and O, and may each independently be substituted with at least one substituent selected from the group consisting of halogen, C₁₋₄ alkyl, and CF₃; and the amino may be substituted at least one substituent selected from the group consisting of H, halogen, CN, C₁₋₆ alkyl, haloC₁₋₃ alkyl or C₁₋₆ alkoxycarbonyl), or R₁ and R₂ may be combined with each other to form 8- to 16-membered heterofused ring (in which the heterofused ring may each independently contain at least one heteroatom selected from the group consisting of N, S and O, and may each independently be substituted with at least one substituent selected from the group consisting of halogen, C₁₋₄ alkyl, and CF₃); and
the linker contains a biocompatible polymer connected to at least one of R₁, R₂, or a ring formed when R₁ and R₂ are combined with each other.

In addition, in another embodiment of the present invention, there is provided a method for preparing an *in vivo* long-acting recombinant coagulation factor VIII, comprising the steps of: (1) introducing, into a host cell, an expression vector that contains nucleotides encoding a coagulation factor VIII, and (2) culturing the host cell in a culture medium supplemented with a derivative of sialic acid metabolite having a first functional group attached thereto, to obtain a coagulation factor VIII to which the sialic acid derivative having the first functional group attached thereto is introduced at the terminus of at least one sugar chain.

In addition, in yet another embodiment of the present invention, there is provided the method for preparing an *in vivo* long-acting recombinant coagulation factor VIII, further comprising the steps of: (3) removing the derivative of the sialic acid metabolite from the culture medium, after carrying out step (2); (4) adding, to the culture medium, a compound having a second functional group and a biocompatible polymer attached thereto, to allow a click reaction; and (5) collecting a coagulation factor VIII to which the biocompatible polymer is attached at the terminus of at least one sugar chain.

### Advantageous Effects of Invention

The *in vivo* long-acting recombinant coagulation factor VIII according to the present invention has a biocompatible polymer, such as polyethylene glycol, conjugated to its sugar chain present on the protein surface, so that immunogenicity of the coagulation factor VIII itself, which is a glycoprotein, can be decreased in the body, and *in vivo* long-acting performance thereof can be improved. In addition, the method for preparing an *in vivo* long-acting recombinant coagulation factor VIII according to the present invention utilizes a normal sugar metabolism process of a host cell of interest in glycoprotein expression using a recombinant DNA technique, thereby having advantages of high efficiency and accuracy in conjugation between the sugar chain and the biocompatible polymer. Therefore, it is possible to provide a modified recombinant coagulation factor VIII in a stable and process-efficient manner, as compared with conventional methods of converting a glycoprotein sugar chain using a sugar conjugation technique.

### Brief Description of Drawings

FIG. 1 illustrates a map of an expression vector used when transfecting scFVIII G4 B3 into a host cell in Example 1.1.
FIG. 2 illustrates results obtained by analyzing, with SDS PAGE, a purification process of scFVIII G4_B3 in Example 1.2. (lane 1: molecular weight markers, lane 2: cell culture medium concentrate, lane 3: VIII select eluate, lane 4: purified scFVIII G4 B3).
FIG. 3 illustrates a cell culture process in Example 2.1 and details of each cycle thereof.
FIGS. 4A and 4B illustrate growth curves and expression levels of scFVIII G4 B3, respectively, for a cell line expressing scFVIII G4 B3 with Ac4ManNAl (N-(4-pentynoyl)-mannosamine-tetraacylated), and a cell line expressing scFVIII G4 B3 without Ac4ManNAl.
FIG. 5 illustrates expression levels of scFVIII G4 B3 for a cell line expressing scFVIII G4 B3 with Ac4ManNAz, and a cell line expressing scFVIII G4 B3 without Ac4ManNAz (N-azidoacetylmannosamine-tetraacylated).
FIG. 6A illustrates results obtained by analyzing, with SDS PAGE, glycoPEGylation reaction products of scFVIII G4 B3 Az and purified scFVIII G4 B3 Az (lane 1: molecular weight markers, lane 2: reaction product of scFVIII G4 B3 Az with 20 kDa DBCO PEG, lane 3: reaction product of scFVIII G4 B3 Az with 30 kDa DBCO PEG, lane 4: reaction product of scFVIII G4 B3 Az with 40 kDa DBCO PEG, lane 5: purified scFVIII G4 B3 Az).
FIG. 6B illustrates results obtained by analyzing, with SDS PAGE, a glycoPEGylation reaction product of scFVIII G4 B3 Az and purified scFVIII G4 B3 Az (lane 1: molecular weight markers, lane 2: purified scFVIII G4 B3 Az, lane 3: reaction product of scFVIII G4 B3 Az with 10 kDa DBCO PEG).
FIG. 6C illustrates results obtained by analyzing, with SDS PAGE, purified versions of scFVIII G4 B3 Az and a glycoPEGylation reaction product of scFVIII G4 B3 Az (lane 1: molecular weight markers, lane 2: purified scFVIII G4 B3 Az, lane 3: purified 20 kDa DBCO PEG-conjugated scFVIII G4 B3 Az, lane 4: molecular weight markers).
FIG. 6D illustrates results obtained by analyzing, with SDS PAGE, a purified version of a glycoPEGylation reaction product of scFVIII G4 B3 Az (lane 1: molecular weight markers, lane 2: purified 10 kDa DBCO PEG-conjugated scFVIII G4 B3 Az).
FIG. 7 illustrates results obtained by analyzing, with SDS PAGE, a product obtained in a case where a click reaction is performed by adding azide-PEG40kDa to scFVIII G4 B3 A1 in the presence of Cu²⁺.
FIG. 8 illustrates a graph showing activity of Factor VIII remaining in the blood over time after intravenous administration of glycoPEGylated FVIII, compared to non-pegylated FVIII.
FIG. 9 illustrates a graph showing activity of Factor VIII remaining in the blood over time after subcutaneous administration of glycoPEGylated FVIII, compared to non-pegylated FVIII.
FIG. 10A illustrates a graph showing activity of Factor VIII remaining in the blood over time after subcutaneous administration of glycoPEGylated FVIII, compared to non-pegylated FVIII.
FIG. 10B illustrates a graph showing activity of Factor VIII remaining in the blood over time after subcutaneous administration of glycoPEGylated FVIII, compared to non-pegylated FVIII.

### Best Mode for Carrying out the Invention

In an aspect of the present invention, there may be provided an *in vivo* long-acting recombinant coagulation factor VIII (Factor VIII, FVIII), comprising a linker which is linked to a sialic acid derivative at the terminus of at least one sugar chain of a coagulation factor VIII, wherein the linker is represented by Formula 1: 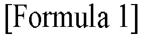 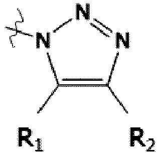 where R₁ and R₂ are each independently C₁₋₈ alkyl, C₆₋₁₂ aryl, C₃₋₆ cycloalkyl, 3- to 10-membered heterocycloalkyl, amino, (amino)C₁₋₆ alkyl, (amino)C₃₋₆ cycloalkyl, (amino)C₆₋₁₂ aryl, (amino)(3- to 10-membered heterocycloalkyl), or 5-to 12-membered heteroaryl (in which the heterocycloalkyl and the heteroaryl may each independently contain at least one heteroatom selected from the group consisting of N, S and O, and may each independently be substituted with at least one substituent selected from the group consisting of halogen, C₁₋₄ alkyl, and CF₃; and the amino may be substituted at least one substituent selected from the group consisting of H, halogen, CN, C₁₋₆ alkyl, haloC₁₋₃ alkyl or C₁₋₆ alkoxycarbonyl), or R₁ and R₂ may be combined with each other to form 8- to 16-membered heterofused ring (in which the heterofused ring may each independently contain at least one heteroatom selected from the group consisting of N, S and O, and may each independently be substituted with at least one substituent selected from the group consisting of halogen, C₁₋₄ alkyl, and CF₃); and
the linker contains a biocompatible polymer connected to at least one of R₁, R₂, or a ring formed when R₁ and R₂ are combined with each other.

The coagulation factor VIII is a glycoprotein in which a sugar chain is linked via a covalent bond to a protein residue. It is known that modifying a sugar chain on the protein surface in glycoproteins may affect protein properties, such as improving water solubility of proteins, protecting proteins from proteases, and determining directionality of proteins in intercellular interactions.

Accordingly, the present inventors used metabolic glycoengineering to conjugate a protein sugar chain to a biocompatible polymer via the linker represented by Formula 1 so that *in vivo* long-acting performance and stability of the coagulation factor VIII are improved, and thus have completed the present invention.

Specifically, in the present invention, the coagulation factor VIII is labeled with a biocompatible polymer using an azide-alkyne cyclization reaction (click reaction) that does not require a catalyst such as copper having cytotoxicity. Unlike other chemical reactions, the chemical reaction is feasible on a precursor even in the body because a reactive group that has been previously introduced into the precursor is azide or alkyne which allows a chemical reaction to occur without any disturbance caused by substances existing in the body.

The biocompatible polymer may be at least one selected from the group consisting of polyethylene glycol (PEG), polysialic acid (PSA), polyoxazoline, and heparosan, with polyethylene glycol being preferred.

In addition, the polyethylene glycol may have a weight average molecular weight of 5 to 40 kDa, 5 to 30 kDa, or 5 to 20 kDa. Multiple conjugation of small-sized polyethylene glycol having a molecular weight of 40 kDa or lower may decrease possibility of PEG toxicity that occurs upon conjugation of polyethylene glycol having a higher molecular weight, further improve the glycoprotein's half-life, and improve bioavailability thereof upon subcutaneous administration.

In addition, the structure of Formula 1 may be at least one selected from the group consisting of Formulas 2 to 18 and isomers thereof. 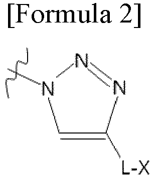 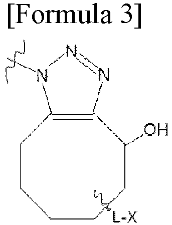 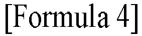 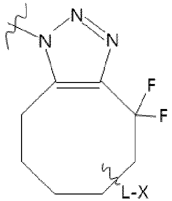 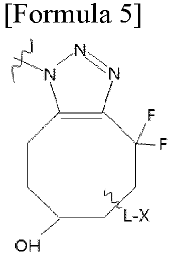 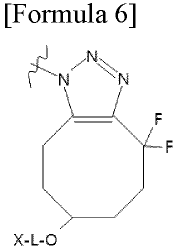 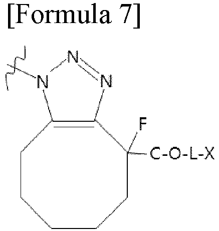 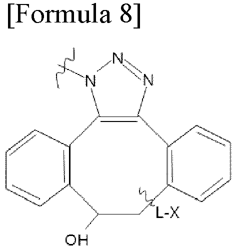 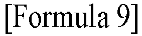 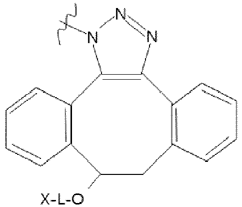 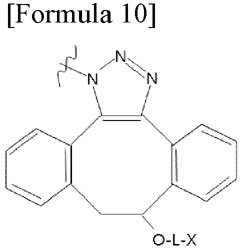 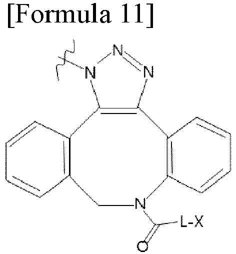 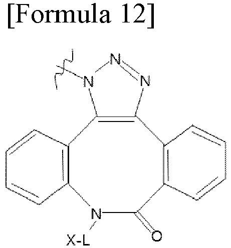 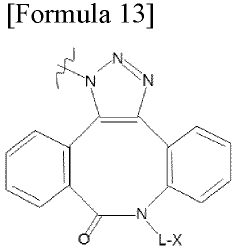 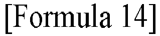 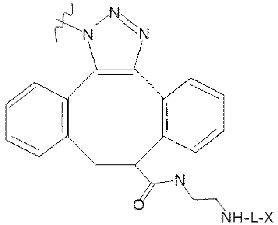 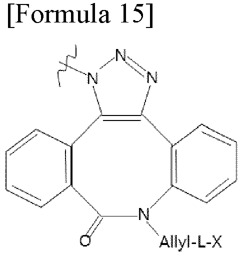 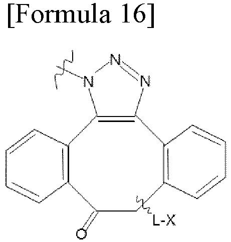 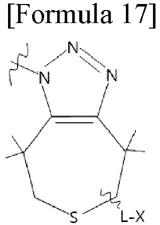 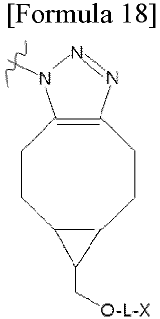 where L may be a single bond, halogen, C₁₋₈ alkyl, C₆₋₁₂ aryl, -CO-,-R₃CONR₃-, -OCONR₃-, or -R₃NCOR₃- (in which R₃ may each independently be selected from H, C₁₋₄ alkyl and C₆₋₁₂ aryl), and X is a biocompatible polymer.

Specifically, the compound may be any one of the compounds represented by Formulas 19 to 26, in which n is an integer from 100 to 3,000.

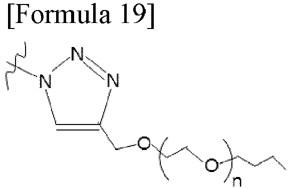

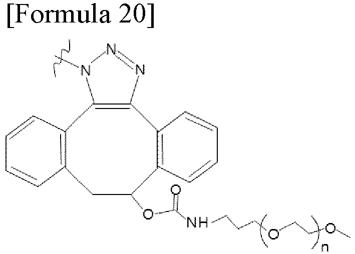

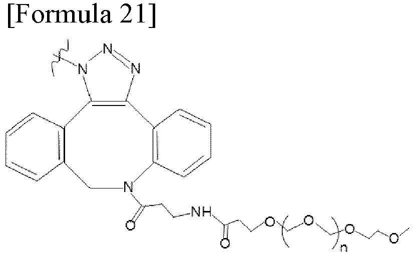

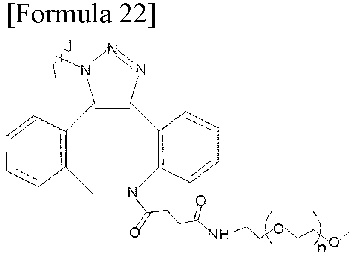

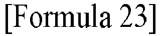

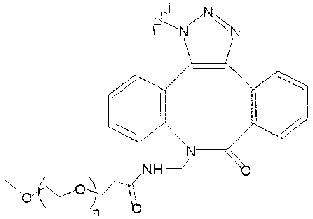

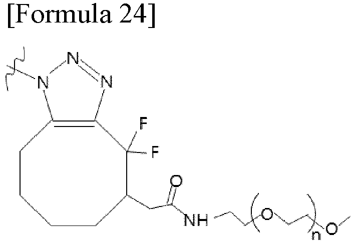

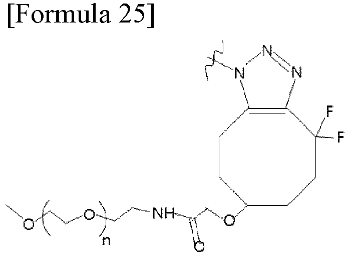

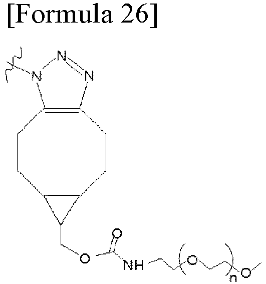

A wild-type Factor VIII protein, which is composed of A1-a1-A2-a2-B-a3-A3-C1-C2 domains, is expressed as a single-chain protein, and then matured by purine to form a heterodimer of 280 kDa containing heavy and light chains in hepatocytes. Factor VIII protein, which may be modified in the present invention, is a normal Factor VIII that may be represented by SEQ ID NO: 1, and is preferably a single-chain form of Factor VIII (scFVIII).

In addition, the Factor VIII protein contains B-domain region that is partially deleted in a contiguous or non-contiguous manner and a3-domain region that is partially deleted; and a single-chain form of Factor VIII which contains the B-domain region that is partially deleted in a contiguous or non-contiguous manner and the a3-domain region that is partially deleted may include any one of SEQ ID NO: 2 (a form obtained from SEQ ID NO: 1 by deletion of amino acid residues 789-1653 of the B domain and a3 domain regions), SEQ ID NO: 3 (a form obtained from SEQ ID NO: 1 by deletion of amino acid residues 833-1653 of the B domain and a3 domain regions), SEQ ID NO: 4 (a form obtained from SEQ ID NO: 1 by deletion of amino acid residues 903-1653 of the B domain and a3 domain regions), SEQ ID NO: 5 (a form obtained from SEQ ID NO: 1 by deletion of amino acid residues 966-1653 of the B domain and a3 domain regions), SEQ ID NO: 6 (a form obtained from SEQ ID NO: 1 by deletion of amino acid residues 903-1653 and 1643-1653 of the B domain and a3 domain regions), SEQ ID NO: 7 (a form obtained from SEQ ID NO: 1 by deletion of amino acid residues 966-1653 of the B domain and a3 domain regions), and SEQ ID NO: 8 (a form obtained from SEQ ID NO: 4 by isoleucine-to-cysteine substitution at amino acid residue 782).

In addition, Factor VIII protein may include variously modified peptides, that is, variants. The modification may be carried out by substitution, deletion, or addition of one or more amino acids within a range that does not alter the function of Factor VIII. These various peptides may be 90%, 91%, 92%, 93%, 94%, 95%, 96%, 97%, 98%, or 99% or more identical to any one of the amino acid sequences of SEQ ID NOs: 2 to 8.

In addition, a sugar chain in the Factor VIII protein, to which the linker is connected, may be included at at least one position of amino acid residues 42, 239, 582, 757, 784, 828, 900, 963, 1001, 1005, 1055, 1066, 1185, 1255, 1259, 1282, 1300, 1412, 1442, 1810, and 2118, based on the amino acid sequence of SEQ ID NO: 1. As such, Factor VIII proteins including single-chain Factor VIII (scFVIII) have many sugar chains on the surface, and thus are suitable for modifying the protein surface by applying metabolic glycoengineering according to the present invention.

In addition, in another aspect of the present invention, there may be provided a method for preparing an *in vivo* long-acting recombinant coagulation factor VIII, comprising the steps of: (1) introducing, into a host cell, an expression vector that contains nucleotides encoding a coagulation factor VIII, and (2) culturing the host cell in a culture medium supplemented with a derivative of a sialic acid metabolite having a first functional group attached thereto.

In step (1), the sequence of the coagulation factor VIII may be any one of the amino acid sequences of SEQ ID NOs: 2 to 8, and the nucleic acid sequence of the nucleotides may be any one of the nucleotide sequences of SEQ ID NOs: 9 to 15. Further, the nucleotides may additionally contain a signal sequence or a leader sequence.

As used herein, the term "signal sequence" refers to a nucleic acid encoding a signal peptide that directs secretion of a fusion protein. The signal peptide is translated in a host cell and then cleaved. Specifically, the signal sequence of the present invention is a polynucleotide encoding an amino acid sequence that initiates movement of a protein through the endoplasmic reticulum (ER) membrane. Signal sequences useful in the present invention include antibody light chain signal sequences, for example, antibody 14.18 (Gillies et al., J. Immunol. Meth 1989. 125: 191-202), antibody heavy chain signal sequences, for example, MOPC141 antibody heavy chain signal sequence (Sakano et al., Nature, 1980. 286: 676-683), and other signal sequences known in the art (for example, Watson et al., Nucleic Acid Research, 1984. 12: 5145-5164).

Characteristics of signal peptides are well known in the art. The signal peptides typically contain 16 to 30 amino acid residues and may contain greater or fewer amino acid residues. Typical signal peptides consist of three regions, that is, a basic N-terminal region, a central hydrophobic region, and a more polar C-terminal region.

The central hydrophobic region contains 4 to 12 hydrophobic residues that immobilize a signal sequence across a membranous lipid bilayer during movement of an immature polypeptide. After the initiation, the signal sequence is cleaved within the lumen of ER by cellular enzymes, commonly known as signal peptidases. Here, the signal sequence may be a signal sequence of tissue plasminogen activator (tPa), a signal sequence of Herpes simplex virus glycoprotein D (HSV gD), or a secretion signal sequence of growth hormone. Preferably, a secretion signal sequence used in higher eukaryotic cells including mammals and the like may be used. In addition, for the secretion signal sequence of the present invention, a signal sequence included in wild-type IL-7 may be used or may be substituted with a codon having a high expression frequency in a host cell and used.

In addition, the expression vector is a vector that can be introduced into a host cell and can be recombined with and inserted into the host cell's genome. The vectors include linear nucleic acids, plasmids, phagemids, cosmids, RNA vectors, viral vectors, and analogs thereof. Examples of the viral vectors include, but are not limited to, retroviruses, adenoviruses, and adeno-associated viruses. In addition, the plasmids may contain a selection marker such as an antibiotic resistance gene, and plasmid-containing host cells may be cultured under selective conditions.

In addition, as used herein, the term "host cell" refers to a prokaryotic or eukaryotic cell into which a recombinant expression vector can be introduced. As used herein, the terms "transduced", "transformed", and "transfected" refer to introduction of a nucleic acid (for example, a vector) into cells using techniques known in the art. Appropriate host cells may be used for expression and/or secretion of Factor VIII proteins by being transduced or transfected with the nucleotide sequences of the present invention. Preferred host cells that can be used in the present invention include Escherichia coli, immortal hybridoma cells, NS/0 myeloma cells, 293 cells, Chinese hamster ovary cells (CHO cells), HeLa cells, human amniotic fluid derived cells (CapT cells), or COS cells. For example, the most preferred host strain used to express a Factor VIII protein in the present invention is CHO-S, and the genes and methods of using the same are known in the art.

In addition, in step (2), supplementation with the derivative of a sialic acid metabolite having a first functional group attached thereto when culturing the host cell is made so as to cause the sialic acid derivative having the first functional group attached thereto to be introduced to the terminus of a sugar chain of the coagulation factor VIII via the sialic acid metabolic pathway of the host cell protein. That is, in step (2), the coagulation factor VIII can be modified with the sialic acid derivative having the first functional group attached thereto.

Here, the first functional group may be an azide group or alkyne group. In addition, the derivative of sialic acid metabolite may contain an acetyl group, or an alkyl group such as an ethyl group and a propyl group in order to be efficiently delivered into cells. The alkyl group forms an ester bond with a hydroxyl group of the sialic acid metabolite having a first functional group attached thereto, to facilitate cell permeation thereof; and after the cell permeation, the alkyl group is removed by intracellular esterases. Thus, the alkyl group does not prevent the supplemented sialic acid metabolite from replacing part of the sugar chain.

In addition, in step (2), the derivative of a sialic acid metabolite, which can replace the sialic acid metabolite (an intermediate substance in the sialic acid biosynthetic pathway of the coagulation factor VIII) may be at least one compound selected from the group consisting of Formulas 27 to 32 and isomers thereof:

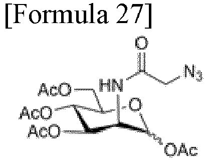

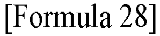

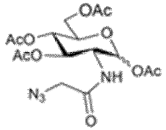

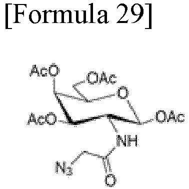

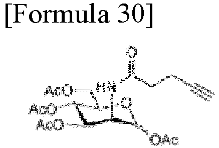

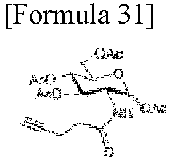

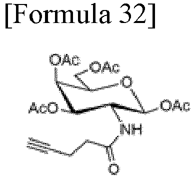

Meanwhile, in step (2), the derivative of the sialic acid metabolite may be supplemented in a concentration of 0.1 to 300 uM (micro mole) when the animal cell-derived host cell has a cell concentration of 1.0 × 10⁵ or higher. Since the method according to the present invention utilizes the cell's sugar metabolism process, the sialic acid metabolite having a first functional group attached thereto may replace not only part of the target coagulation factor VIII, but also part of metabolites resulting from metabolism associated with all glycoproteins in the host cell. Therefore, in order to introduce, to the coagulation factor VIII, the first functional group at a high rate for the purpose of sugar chain modification, it is preferred that the derivative of sialic acid metabolite is supplemented when the host cell has a high cell concentration, preferably a cell concentration of 1.0 × 10⁵ or higher.

It is preferable that the host cell is cultured as follows. During a growth phase of the host cell, culture thereof is performed without supplementation with the derivative of sialic acid metabolite; after a certain level of cell growth is achieved, the cell culture condition is changed to a condition under which the cell growth rate is decreased and the cell survival is maintained; and then the host cell is cultured in a culture medium supplemented with the derivative of sialic acid metabolite having a first functional group. The sialic acid metabolite, which is supplemented at a time when growth of the host cell is decreased, can be introduced to the sialic acid at the sugar chain of a target protein at a higher rate than when the cell is growing.

Here, as a condition under which the cell growth rate is decreased, there is a method of adjusting the temperature to a low temperature or limiting nutrient components of the culture medium. Specifically, when the culture temperature is changed from 37°C during the cell growth phase to 29°C to 35°C at the time of supplementing the derivative of sialic acid metabolite, the cell growth may be slowed, whereas the expression of the target protein (coagulation factor VIII) may be continued for 10 days or longer. In addition, it is possible to decrease the concentration of nutrients such as biological growth factors, vitamins, and essential amino acids in the cell culture medium. In addition, in case of subjecting a sugar chain of Factor VIII to metabolic labeling, a substance such as butyric acid is added to affect the cell cycle, so that the cell growth is stopped and the expression of Factor VIII is maintained as it is, thereby increasing a rate of the sialic acid derivative having a first functional group to be introduced to the sugar chain of Factor VIII.

In addition, the method may further comprise the steps of: (3) removing the derivative of the sialic acid metabolite from the culture medium, after purifying, from step (2), the coagulation factor VIII whose sugar chain contains the sialic acid derivative having a first functional group attached thereto; (4) adding, to the culture medium, a compound having a second functional group and a biocompatible polymer attached thereto, to allow a click reaction to proceed; and (5) collecting a coagulation factor VIII to which the biocompatible polymer is attached at the terminus of at least one sugar chain.

In order to allow the first functional group of the sialic acid derivative in the coagulation factor VIII to react with the second functional group, it is necessary to remove the derivative of sialic acid metabolite which is present in excess in the culture medium.

Here, the first functional group may be an azide group or alkyne group. In addition, the second functional group is an alkyne group or a cycloalkyne group when the first functional group is an azide group; and the second functional group may be an azide group when the first functional group is an alkyne group.

Specifically, in step (4), conjugation of the biocompatible polymer may be performed using a click reaction for the sialic acid derivative having an azide group or alkyne group which has been introduced to a sugar chain of the target protein according to step (2) in the method of the present invention. For example, when the derivative of sialic acid metabolite having an azide group attached thereto is used in step (2), a compound having an alkyne group or a cycloalkyne group and a biocompatible polymer attached thereto may be used in step (4); and when the derivative of sialic acid metabolite having an alkyne group attached thereto is used in step (2), a compound having an azide group and a biocompatible polymer attached thereto may be used in step (4).

In step (4), the compound having a second functional group and a biocompatible polymer attached thereto is preferably at least one compound selected from the group consisting of Formulas 33 to 49 and isomers thereof: 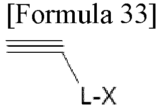 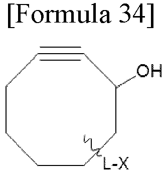 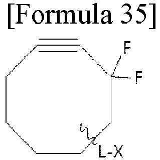 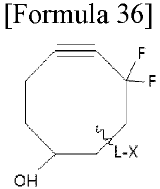 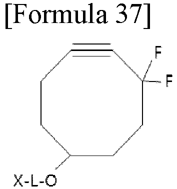 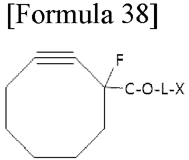 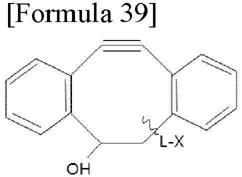 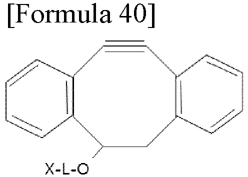 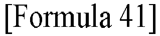 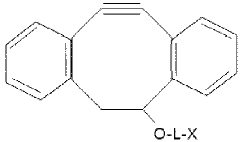 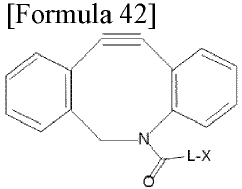 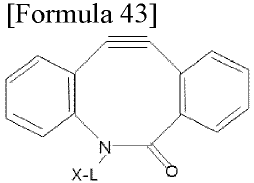 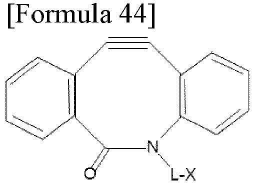 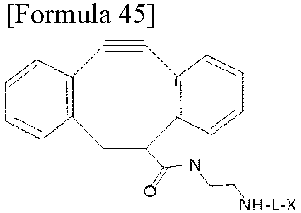 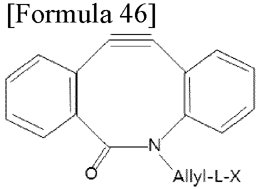 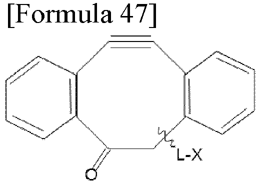 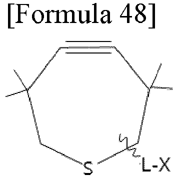 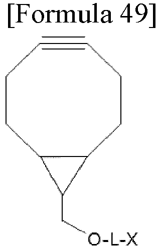 where L may be a single bond, halogen, C₁₋₈ alkyl, C₆₋₁₂ aryl, -CO-,-R₃CONR₃-, -OCONR₃-, or -R₃NCOR₃- (in which R₃ may each independently be selected from H, C₁₋₄ alkyl and C₆₋₁₂ aryl), and X is a biocompatible polymer.

In addition, the coagulation factor VIII collected in step (4) contains a linker represented by Formula 1 which is linked to the sialic acid derivative at the terminus of at least one sugar chain, and specific properties or examples thereof are as described above for the *in vivo* long-acting recombinant coagulation factor VIII according to the present invention: 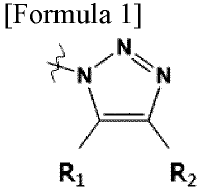 where R₁ and R₂ are each independently C₁₋₈ alkyl, C₆₋₁₂ aryl, C₃₋₆ cycloalkyl, 3- to 10-membered heterocycloalkyl, amino, (amino)C₁₋₆ alkyl, (amino)C₃₋₆ cycloalkyl, (amino)C₆₋₁₂ aryl, (amino)(3- to 10-membered heterocycloalkyl), or 5-to 12-membered heteroaryl (in which the heterocycloalkyl and the heteroaryl may each independently contain at least one heteroatom selected from the group consisting of N, S and O, and may each independently be substituted with at least one substituent selected from the group consisting of halogen, C₁₋₄ alkyl, and CF₃; and the amino may be substituted at least one substituent selected from the group consisting of H, halogen, CN, C₁₋₆ alkyl, haloC₁₋₃ alkyl or C₁₋₆ alkoxycarbonyl), or R₁ and R₂ may be combined with each other to form 8- to 16-membered heterofused ring (in which the heterofused ring may each independently contain at least one heteroatom selected from the group consisting of N, S and O, and may each independently be substituted with at least one substituent selected from the group consisting of halogen, C₁₋₄ alkyl, and CF₃); and
the linker contains a biocompatible polymer connected to at least one of R₁, R₂, or a ring formed when R₁ and R₂ are combined with each other.

### Mode for the Invention

Hereinafter, the present invention will be described in more detail by way of examples. However, the following examples are only for illustrating the present invention, and the scope of the present invention is not limited thereto. Specifically, the technology of the present invention will be described for scFVIII G4 B3 that is one scFVIII of various scFVIII's produced in a patent application (PCT/KR2017/006633) filed by the present inventor(s) prior to this patent application. However, the reason why one scFVIII is selected in describing the technology of the present invention is not because the selected scFVIII has particular advantages, but because the technical description to be presented is made clearer merely as an embodiment.

### Example 1. Preparation of recombinant single-chain (sc) Factor VIII

### Example 1.1. Production of scFVIII-expressing cell line

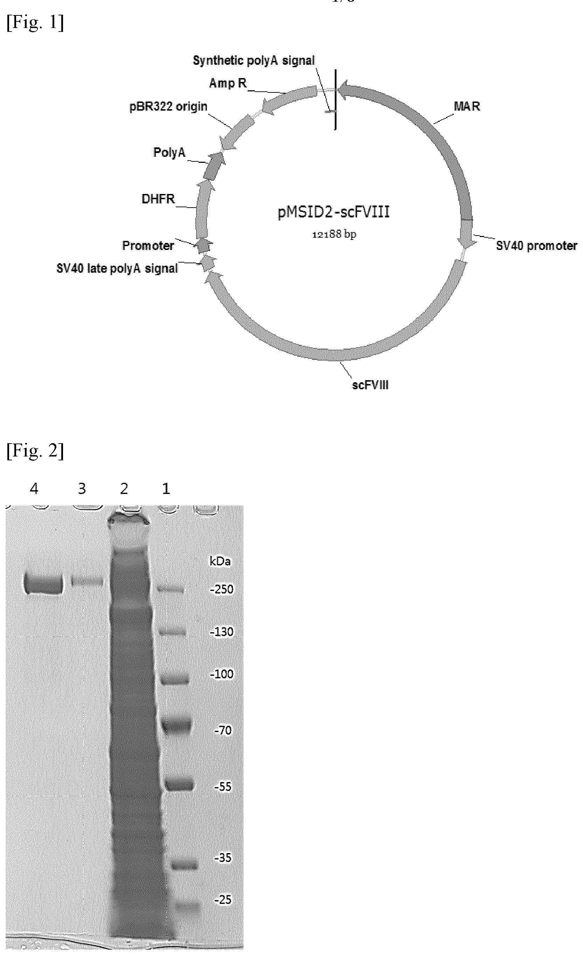

A vector (*see* FIG. 1), which expresses a DNA nucleotide sequence (the nucleotide sequence of SEQ ID NO: 15) encoding scFVIII G4 B3 (the amino acid sequence of SEQ ID NO: 8) described in a patent application (PCT/KR2017/006633) filed by the present inventors, was used to transform a CHO-S cell line. After the transformation, the CHO-S cell line was passaged in the presence of methotrexate (MTX) of 500 mM or higher, and a scFVIII G4 B3-high-expressing cell line (CHO-S G4 B3) was selected. The selected cell line, CHO-S G4 B3 clone, was propagated in CD FortCHO cell culture medium, and then stored in a nitrogen tank.

### Example 1.2. Culture and purification of scFVIII

The selected scFVIII G4 B3-high-expressing cell line, CHO-S G4 B3, was added to CD FortCHO medium so that the number of cells was 3 × 10⁵/mL, and then cell culture was performed for 4 days. After the cell culture, the culture medium was collected and the expressed scFVIII G4 B3 was purified. In order to purify scFVIII from the culture medium, 5 M NaCl solution was added to the culture medium, in which scFVIII G4 B3 was expressed, so that NaCl has a final concentration of 1.0M, and then the culture medium was passed through a 0.2 µm membrane filter to remove fine suspended matters. Then, the filtrate was injected into the FVIII select resin (GE Healthcare) equilibrated with FVIII select equilibrium solution (20 mM histidine, 5 mM CaCl₂, 1 M NaCl, 0.02% Tween®80, pH 7.0) so that scFVIII G4 B3 was attached thereto. Then, VIII select equilibration buffer was used to remove impurities, and VIII select elution buffer (20 mM histidine, 5 mM CaCl₂, 0.9 M arginine, 45% propylene glycol, 0.02% Tween®80, pH 6.5) was used to perform elution (using affinity chromatography). Next, the VIII select eluate was diluted 5 times or higher with an equilibration buffer solution for ion exchange chromatography (20 mM histidine, 5 mM CaCl₂, 0.02% Tween®80, pH 7.0), and then injected into SP Sepharose fast flow ion exchange chromatography resin (GE Healthcare) equilibrated with the equilibrium solution for ion exchange chromatography.

Thereafter, the equilibrium solution for ion exchange chromatography was used to remove impurities, and an ion exchange chromatography eluate (20 mM histidine, 5 mM CaCl₂, 0.02% Tween®80, 400 mM NaCl, pH 6.5) was used to elute pure scFVIII G4 B3. FVIII activity of the purified scFVIII G4 B3 was quantified by the CS assay described in Example 1.3. below. The results of SDS PAGE performed to analyze intermediate substances at respective purification stages are as illustrated in FIG. 2.

### Example 1.3. Measurement of scFVIII activity

The purified scFVIII G4 B3 was measured for FVIII activity by CS method. To measure activity by the chromogenic method, a chromogenic assay kit sold by CHROMOGENIX was used with an endpoint method. The test was conducted using the test method provided by the manufacturer which was modified as follows so as to be suitable for this test. Specifically, samples were diluted to fall within a standard range (0.25 to 1 IU/mL) using 1× dilution buffer. Standards (calibration plasma) were prepared using 1× dilution buffer to have concentrations of 0, 0.25, 0.5, 1 IU/mL. 10 mL each of the thus prepared samples and standards was diluted with 790 mL of 1x dilution buffer. Then, 50 uL each of the dilutions was dispensed into a 96-well plate, and left to stand at 37°C for 5 minutes. Using an automatic dispenser pipette, 50 uL each of agent samples was dispensed into each well and reaction was allowed to occur at 37°C for 2 minutes. 50 uL of substrate was dispensed into each well, and then left to stand at 37°C for 2 minutes before development. To the developed sample was added 50 uL of 2% citric acid, to stop the reaction. Absorbance was measured at a wavelength of 405 nm to draw a linear calibration curve. Then, the sample's absorbance was put into the calibration curve to measure activity of the sample, and the results are shown in Table 1 below. The purified scFVIII G4_B3 had activity of about 7,000 IU/mg, which is similar to that of a commercially available recombinant FVIII (Advate®) intended to treat hemophilia. Thus, it was determined that the purified scFVIII G4 B3 has a level of activity sufficient to be used as a therapeutic agent for hemophilia.

**[Table 1]**

| Sample substance | Specific activity (IU/mg) |
|---|---|
| scFVIII G4_B3 | 7,166 |
| FVIII | 6,000 to 7,000 |

### Example 2. Labeling of scFVIII using glyco-metabolic labeling

### Example 2.1. Labeling of scFVIII with Ac4ManNAl

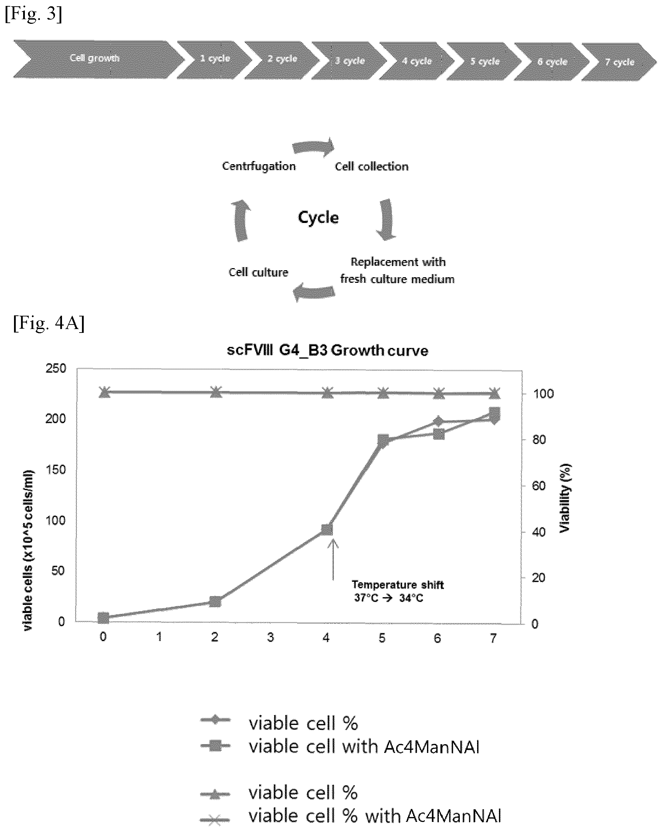

CHO-S scFVIII G4 B3 cell line was suspended in cell culture medium (CD FortiCHO) to a cell concentration of 0.3 to 3.0 × 10⁶, and then cell culture was performed for 4 days with stirring at 140 rpm under a condition of 37°C and 5% CO₂. When the cell concentration reached a certain level, Ac4ManNAl (N-(4-pentynoyl)-mannosamine-tetraacylated) was supplemented to a final concentration of 100 uM, and then cell culture was performed for 24 hours with stirring at 140 rpm under a condition of 31°C and 5% CO₂. After completion of the cell culture, the cell culture medium was centrifuged at 1,000 rpm to remove the culture medium, and then the collected cells were suspended in cell culture medium (CD FortiCHO) supplemented with Ac4ManNAl to a final concentration of 100 uM, so that the cell concentration was adjusted to a level of about 0.1 to 1.5 × 10⁷. Then, culture was performed for 24 hours with stirring at 140 rpm under a condition of 31°C and 5% CO₂. Subsequently, a cell culture cycle was allowed to proceed three or more times (pseudo-perfusion cycle), each cycle involving a process in which culture was performed under the same condition as above while replacing, every 24 hours, the cell culture medium with fresh cell culture medium supplemented with Ac4ManNAl to a final concentration of 100 uM. The cell culture process is illustrated in FIG. 3.

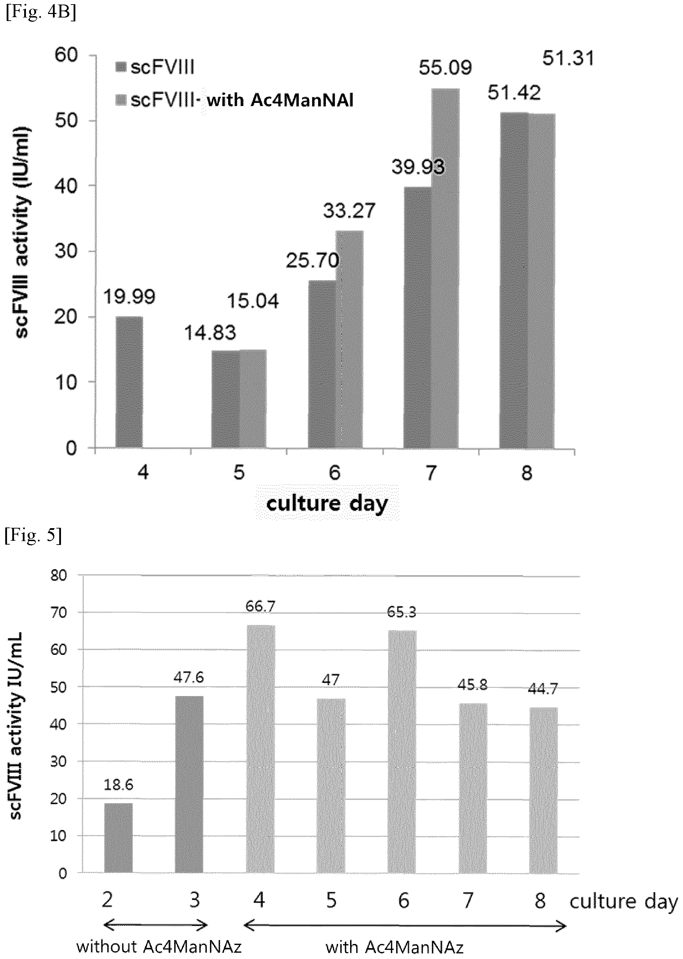

In order to determine an effect of supplementation of the medium with Ac4ManNAl on the growth of scFVIII G4 B3 cells and the expression of scFVIII G4 B3, after the initial 4-day cell growth period, subsequent cell culture cycles were allowed to proceed with cell culture medium not supplemented with Ac4ManNAl, so that comparison on the cell growth and the expression of scFVIII G4 B3 was made in terms of FVIII activity. Supplementation with Ac4ManNAl at 100 uM had no significant effect on the growth of CHO-S scFVIII G4 B3 cells and the expression of scFVIII G4 B3. The initial 4-day culture without Ac4ManNAl caused the cell concentration to reach a level of about 1.0 × 10⁷ cells/mL, and the subsequent culture performed after supplementation with Ac4ManNAl at 100 uM caused the cell concentration to reach a level of 1.7 × 10⁷ cells/mL. Thereafter, the cell concentration was maintained at a constant level. As shown in the growth curves of FIG. 4A and the expression level graphs of FIG. 4B, it was identified that scFVIII G4 B3 was expressed at a similar level regardless of the supplementation with Ac4ManNAl at 100 uM. Accordingly, it can be seen that the supplementation with Ac4ManNAl in a concentration range used for culture had no significant effect on the growth of the scFVIII G4 B3-expressing cell line and the expression level of scFVIII G4 B3.

### Example 2.2. Labeling of scFVIII with Ac4ManNAz

The ScFVIII G4 B3 cells were cultured in a similar manner while being supplemented with Ac4ManNAz (N-azidoacetylmannosamine-tetraacylated) at 100 uM in place of Ac4ManNAl in the same method as in Example 2.1. After the initial 3-day culture, during each cycle of passage, culture was performed with passage culture medium whose volume corresponds to 50% of that of the passage culture medium in Example 2.1., to maintain a high cell concentration. Similar to Ac4ManNAl, the supplementation with Ac4ManNAz at 100uM had no significant effect on the cell growth and the expression of scFVIII. Regarding the cell concentration, starting from day 4 after the culture was shifted to 31°C, the culture medium was replaced daily with the equal volume of fresh medium as in Example 2.1. The scFVIII G4 B3 cells were maintained at a level of 1.5 × 10⁷ or higher until the end of the culture; and the cell viability was maintained at 90% or higher until the end of the culture. As illustrated in FIG. 5, the expression level of scFVIII G4 B3 was similar to that obtained in a case where the scFVIII G4 B3 cells are cultured while being supplemented with Ac4ManNAl in Example 2.1. In conclusion, it was found that the supplementation with Ac4ManNAz at a concentration used for culture had neither a significant effect on the cell growth of the scFVIII G4 B3 cell line nor the expression of scFVIII G4 B3.

### Example 3. Preparation of modified scFVIII

### Example 3.1. Purification of scFVIII labeled with azide or alkyne group and activity measurement thereof

scFVIII G4 B3Az (labeled with Ac4ManNAz) and scFVIII G4 B3Al (labeled with Ac4ManNAl), which had been labeled with azide and alkyne groups, respectively, using Ac4ManNAz and Ac4ManNAl, were purified in the same method as described in Example 1.2. above for scFVIII G4 B3. Activity measurement of the purified scFVIII G4 B3Az and scFVIII G4 B3Al was also performed in the same method as in Example 1.3. above, and the results are shown in Table 2 below. CS activity measurement shows that specific activity of the purified scFVIII G4 B3Az and scFVIII G4 B3Al was similar to that of scFVIII G4 B3 as shown in Table 2. From these results, it was found that labeling of scFVIII G4 B3 with Ac4ManNAz or Ac4ManNAl through glycol-metabolic labeling had no effect on activity of scFVIII G4.

**[Table 2]**

| Sample substance | Specific activity (IU/mg) |
|---|---|
| scFVIII G4 B3 | 7,166 |
| scFVIII G4 B3Az (scFVIII G4 B3 labeled with Ac4ManNAz) | 6,600 |
| scFVIII G4 B3Al (scFVIII G4 B3 labeled with Ac4ManNAl) | 5,860 |

### Example 3.2. Preparation of glycoPEGylated scFVIII through click chemistry

### Example 3.2.1. Preparation of glycoPEGylated scFVIII with Cu²⁺ free click chemistry

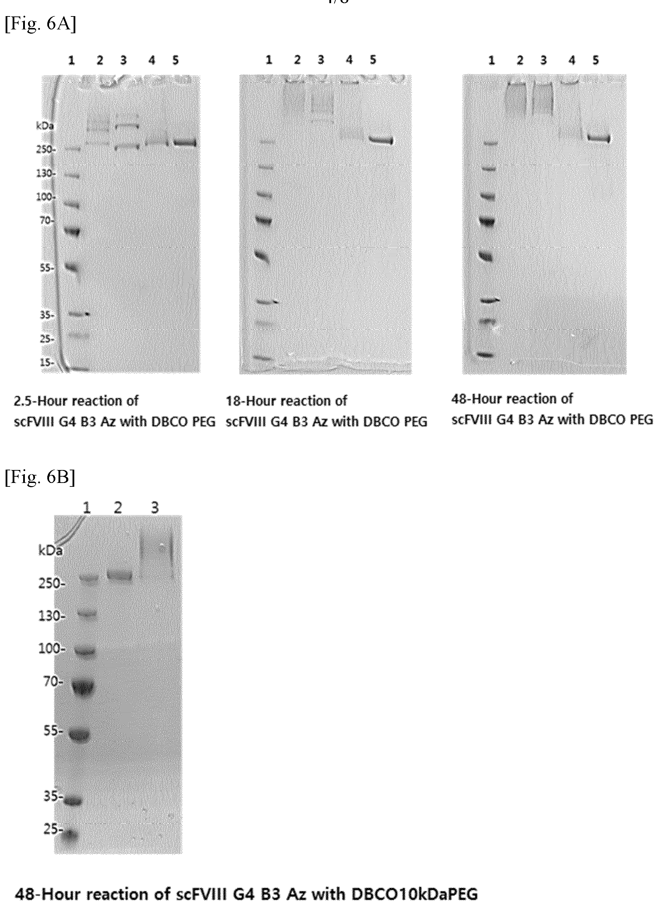

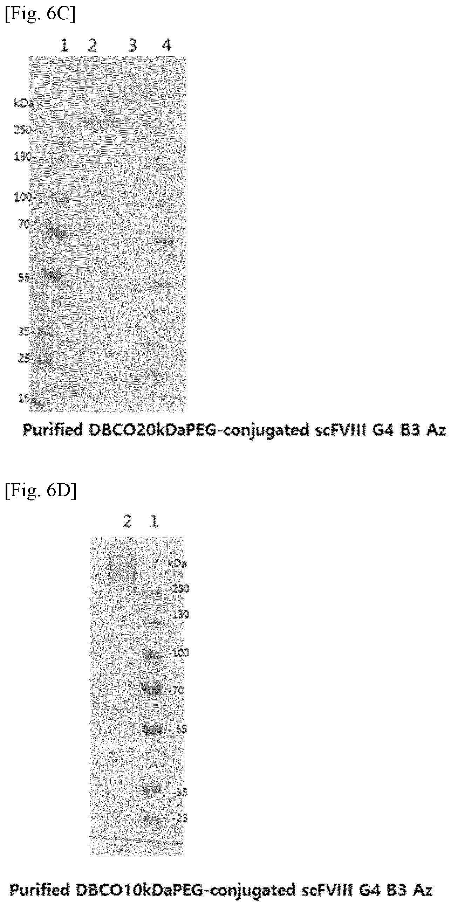

After exchange into a conjugation buffer (20 mM HEPES, 150 mM NaCl, 5 mM CaCl₂, 0.01% (w/v) Tween® 80, pH 7.5), to scFVIII G4 B3Az purified in Example 3.1. was added DBCO-PEG10kDa, DBCO-PEG20kDa, DBCO-PEG30kDa so that each molar ratio of DBCO-PEG (10 kDa, 20 kDa, 30kDa) to scFVIII G4 B3Az is 100:1. Then, reaction was allowed to proceed at 4°C for 48 hours, and thus glycoPEGylated scFVIIIs (DBCOPEG10kDa-scFVIII G4 B3Az, DBCOPEG20kDa-scFVIII G4 B3Az, DBCO-PEG30kDa-scFVIII G4 B3Az) were produced. As illustrated in FIGS. 6A and 6B, it was identified that most of the scFVIII G4 B3 Az, which is scFVIII whose sugar has been modified with Ac4ManNAz, is conjugated with DBCO PEG. In addition, it was identified that consistent with the fact that a plurality of sialic acid molecules in a sugar chain of scFVIII are modified with Ac4ManNAz, multiple conjugation occurs to form a mixture of polymer conjugates. The above DBCOPEG10kDa-, DBCOPEG20kDa-, and DBCOPEG30kDa-scFVIII's were purified by the same method. Specifically, the reaction conjugates were injected into a DEAE Sepharose fast flow ion exchange resin equilibrated with an equilibration buffer (20 mM HEPES, 5 mM CaCl₂, 0.02% (w/v) Tween®80) for attachment, and then unPEGylated scFVIII G4 B3Az was removed with a washing buffer (20 mM HEPES, 5 mM CaCl₂, 0.02% (w/v) Tween®80). Then, glycoPEGylated scFVIII G4 B3Az was eluted with an elution buffer (20 mM HEPES, 5 mM CaCl₂, 500 mM NaCl, 0.02% (w/v) Tween®80). The glycoPEGylation reaction products and purified glycoPEGylated scFVIII's were checked through SDS PAGE, and the results are illustrated in FIG. 6C. Activity of the eluted glycoPEGylated scFVIII's (DBCOPEG10kDa-scFVIII, DBCOPEG20kDa-scFVIII, DBCOPEG30kDa-scFVIII) was measured by the CS method as described in Example 1.3. above. As shown in Table 3, their activity was not decreased as compared with scFVIII G4_B3Az before glycoPEGylation. As a result, it was found that labeling with Ac4ManNAz metabolism and subsequent glycoPEGylation do not decrease activity of scFVIII G4 B3Az.

**[Table 3]**

| Sample substance | Specific activity (IU/mg) |
|---|---|
| scFVIII G4_B3Az | 6,600 |
| DBCOPEG10kDa-scFVIII G4_B3Az | 6,700 |
| DBCOPEG20kDa-scFVIII G4_B3Az | 6,100/5,800 |
| DBCOPEG30kDa-scFVIII G4_B3Az | 5,000 |

### Example 3.2.2. Production of glycoPEGylated single-chain FVIII using Cu²⁺ click chemistry

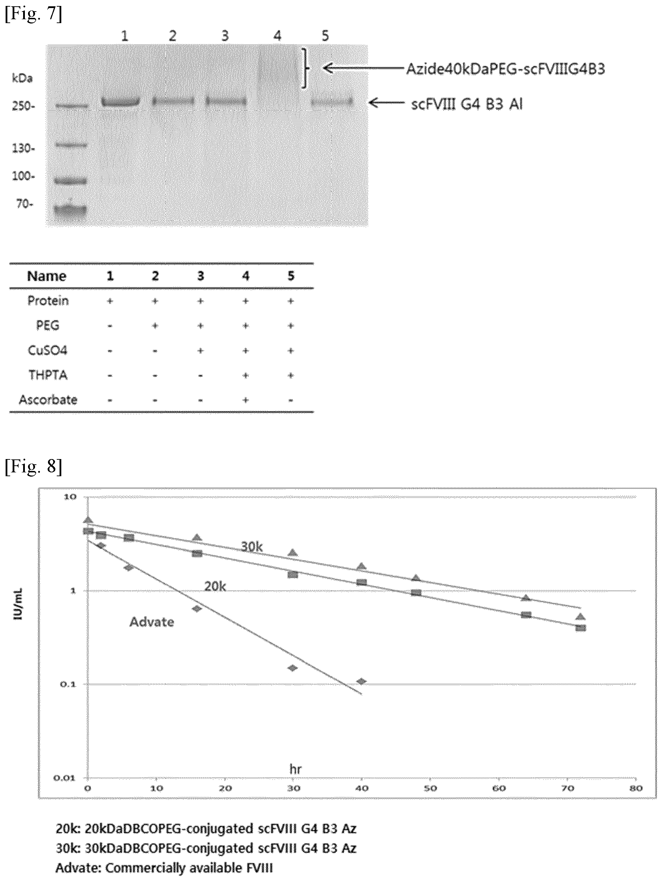

After exchange into a conjugation buffer (20 mM HEPES, 150 mM NaCl, 5 mM CaCl₂, 0.01% (w/v) Tween®80), to scFVIII G4 B3 Al purified in Example 3.1. was added azide-PEG40kDa (Nanocs, PG1-AZ-40k) so that a molar ratio of azide-PEG40kDa to scFVIII G4 B3 Al is 1:100. To initiate conjugation reaction between scFVIII G4 B3 Al and azide-PEG40kDa, Cu²⁺ and THPTA were added so that Cu²⁺ had a final concentration of 0.1 mM and THPTA had a final concentration of 0.5 mM; and then ascorbate was added so that ascorbate has a final concentration of 5 mM. Then, reaction was allowed to proceed at room temperature for 24 hours. The conjugation reaction product was checked through SDS PAGE, and the results are illustrated in FIG. 7. Most of scFVIII G4 B3 Al's were conjugated. This means that most of the sugar chains of scFVIII G4 B3 were modified with alkyne by way of Ac4ManNAl. After the 24-hour conjugation reaction, activity of the conjugation reaction product was measured by the CS method as described in Example 1.3. It was identified that the conjugation product lost 50% or higher of activity as compared with the activity of FVIII in scFVIII G4 B3 Al before the conjugation reaction. These results are presumed to be because free radicals, generated by reaction of Cu²⁺ and ascorbate which have been added for the conjugation reaction between alkyne-labeled scFVIII B3 G4Al and azide-PEG40kDa, resulted in denaturation of the scFVIII G4 B3 Al protein.

### Example 4 Evaluation of in vivo long-acting performance of scFVIII having polyethylene glycol-labeled sugar chain

### Example 4.1. Pharmacokinetics after administration by intravenous injection

Hemophilia mice were administered by intravenous injection of scFVIIIs (glycoPEGylated scFVIII) labeled with polyethylene glycol which had been prepared through Example 3.1. and Example 3.2.1. Then, pharmacokinetics thereof was measured. DBCOPEG20kDa-scFVIII G4 B3 Az, DBCOPEG30kDa-scFVIII G4 B3 Az, and the positive control Advate® were administered by intravenous injection through the tail vein of 13-week-old female hemophilia mice (B6; 129S4-F8tm1Kaz/J (FVIII-knock-out)). Then, activity of FVIII remaining in the blood was measured up to 72 hours. DBCOPEG20kDa-scFVIII G4 B3 Az, DBCOPEG30kDa-scFVIII G4 B3 Az, and Advate® were respectively administered through intravenous tail vein injection at a dose of 200 IU/kg; and blood was collected from the retro-orbital venous sinus of each mouse at fixed time points (2 min, 6 hr, 16 hr, 30 hr, 40 hr, 48 hr, 64 hr, 72 hr). To the collected blood was added an anticoagulant (3.2% sodium citrate) so that the anticoagulant had a final concentration of 10% (v/v). Then, centrifugation was performed to obtain plasma. Quantification of the administered substance and quantification of activity of FVIII remaining in the plasma were performed by the CS measurement method as described in Example 1.3. Activity of FVIII remaining in the plasma, which was collected at the respective time points from the mice in each sample group, is illustrated in FIG. 8. In addition, based on the measurement values of FVIII activity remaining in the plasma, PK parameters were derived therefrom through the non-compartmental analysis (NCA) method using WinNonlin software, and the results are shown in Table 4. The half-life of each of DBCOPEG20kDa-scFVIII G4 B3 Az and DBCOPEG30kDa-scFVIII G4 B3 Az was calculated from the initial 2 minutes to 72 hours based on the average FVIII activity of mice. As compared with Advate®, the half-life was increased 2.8 times (for DBCOPEG20kDa-scFVIII G4 B3 Az) and 3.2 times (for DBCOPEG30kDa-scFVIII G4 B3 Az), respectively. As compared with Advate®, an area under the curve (AUC_{INF}) was also increased by 3.7 times (for DBCOPEG20kDa-scFVIII G4 B3 Az) and 5.1 times (for DBCOPEG30kDa-scFVIII G4 B3 Az), respectively. Accordingly, it was identified that in case of being administered by intravenous injection, the modified Factor VIII protein prepared according to the present invention remained in the body for a long time as compared with the existing FVIII, and exhibits a long-acting effect correspondingly.

**[Table 4]**

| Pharmacokinetic parameters | Advate® | | | DBCOPEG20kDa-scFVIII G4 B3 Az | | | DBCOPEG30kDa-scFVIII G4 B3 Az | | |
|---|---|---|---|---|---|---|---|---|---|
| T_{1/2}, terminal (hr) | 7.6 | ± | 0.8 | 21.1 | ± | 5.5 | 25.6 | ± | 1.9 |
| Cmax (IU/mL) | 3.9 | ± | 2.1 | 4.3 | ± | 0.0 | 5.7 | ± | 0.3 |
| AUCₗₐₛₜ (hr^{∗}IU/mL) | 34.0 | ± | 2.2 | 120.3 | ± | 12.2 | 153.7 | ± | 11.7 |
| AUC_{INF_ obs} (hr^{∗}IU/mL) | 35.0 | ± | 2.4 | 131.1 | ± | 17.4 | 178.9 | ± | 17.6 |
| AUC%_{_Extrap} (%) | 2.8 | ± | 0.9 | 7.9 | ± | 3.0 | 9.4 | ± | 1.7 |
| CL (mL/hr/kg) | 3.6 | ± | 0.3 | 1.0 | ± | 0.1 | 0.7 | ± | 0.1 |
| MRT_{INF} (hr) | 9.6 | ± | 1.1 | 28.7 | ± | 2.8 | 31.7 | ± | 2.9 |

### Example 4.2.1. Pharmacokinetics after administration by subcutaneous injection

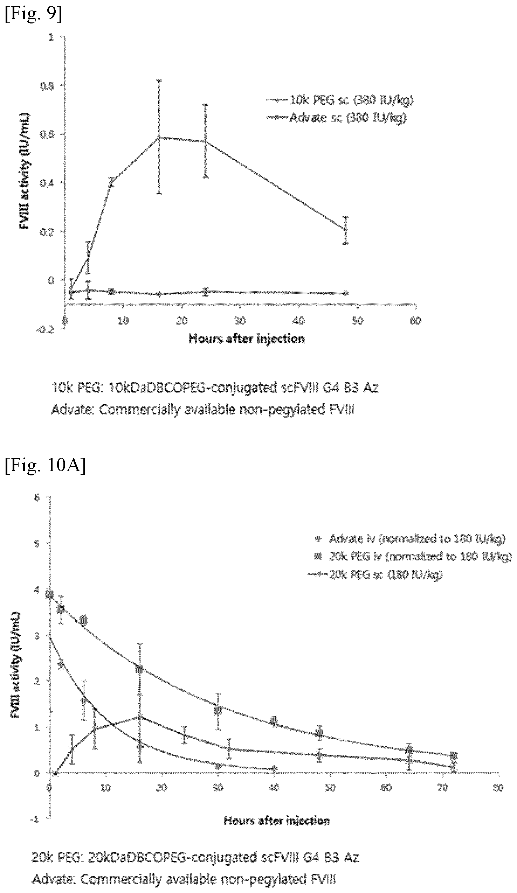

A pharmacokinetic test was performed in which hemophilia mice are administered by subcutaneous injection of glycoPEGylated scFVIIIs produced through Example 3.1. and Example 3.2.1. 13-Week-old female hemophilia mice (B6; 129S4-F8tm1Kaz/J (FVIII-knock-out)) were administered by subcutaneous injection of DBCOPEG10kDa-scFVIII G4 B3 Az, which is glycoPEGylated scFVIII, and the positive control Advate®. Then, activity of FVIII remaining in the blood was measured up to 48 hours. The mice were administered by subcutaneous injection of each sample at a dose of 380 IU/kg based on FVIII activity (based on the titer of an international titer standard for FVIII); and blood was collected from the retro-orbital venous sinus of each mouse at fixed time points (1 hr, 4 hr, 8 hr, 16 hr, 24 hr, 48 hr). To the collected blood was added an anticoagulant (3.2% sodium citrate) so that the anticoagulant has a final concentration of 10% (v/v). Then, centrifugation was performed to obtain plasma, and the plasma was stored frozen until measurement of FVIII activity. Activity of FVIII remaining in the plasma, which was collected at the respective time points from the mice in each sample group, is illustrated in FIG. 9. Based on the measurement values of FVIII activity remaining in the plasma, PK parameters were derived therefrom through the non-compartmental analysis (NCA) method using WinNonlin software, and the results are shown in Table 5 (pharmacokinetic parameters obtained in a case where DBCOPEG10kDa-scFVIII G4 B3 Az is subcutaneously administered). DBCOPEG10kDa-scFVIII G4 B3 Az in each mouse had a terminal half-life which corresponds to a level of 14 to 24 hours, and this was a level that is increased 1.8 to 3.2 times as compared with the half-life of Advate® in Example 4.1. Consistent to the fact that bioavailability of the existing FVIII is less than 1% in a case of being subcutaneously administered in mice (American society of hematology annual meeting (ASH) 2015, Recombinant FVIIIFc-VWF-XTEN demonstrates significant bioavailability following subcutaneous administration in hemophilia A mice), Advate® was present at a level that is not observed in the blood in case of being subcutaneously administered. However, DBCOPEG10kDa-scFVIII G4 B3 Az was observed, up to 48 hours, at a level that is high enough to cause a hemostatic reaction to bleeding in hemophilia mice. This means that DBCOPEG10kDa-scFVIII G4 B3 Az, which is glycoPEGylated scFVIII, is stable in a subcutaneous environment and stable even after entering the bloodstream, thereby exhibiting bioavailability higher than the existing FVIII. As a result, it was found that DBCOPEG10kDa-scFVIII G4 B3 Az, which is glycoPEGylated scFVIII, could be developed as a subcutaneous formulation, unlike the existing FVIII.

**[Table 5]**

| T_{1/2}(hr) | Tₘₐₓ(hr) | Cₘₐₓ (IU/mL) | AUCₗₐₛₜ(hr^{∗}IU/mL) | AUC_{INF_obs} (hr^{∗}IU/mL) |
|---|---|---|---|---|
| 19.2 | 18.7 | 0.618 | 18.1 | 21.7 |

### Example 4.2.2. Pharmacokinetics after administration by subcutaneous injection

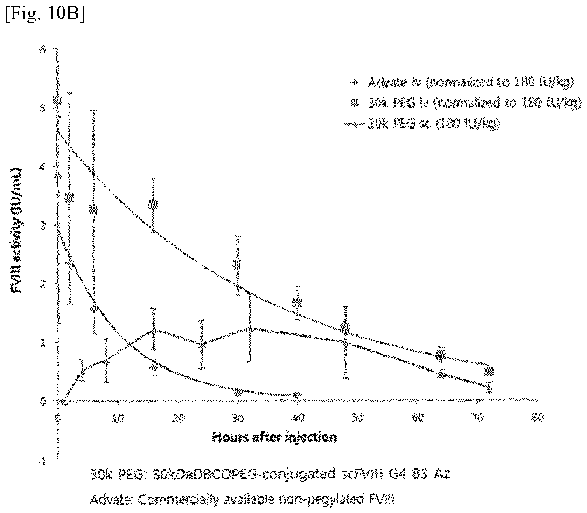

Hemophilia mice were administered by intravenous injection of glycoPEGylated scFVIII produced through Example 3.1. and Example 3.2.1. Then, pharmacokinetics thereof was measured. 13-Week-old female hemophilia mice (B6; 129S4-F8tm1Kaz/J (FVIII-knock-out)) were administered by subcutaneous injection of DBCOPEG20kDa-scFVIII G4 B3 Az and DBCOPEG30kDa-scFVIII G4 B3 Az respectively, which are glycoPEGylated scFVIIIs. Then, activity of FVIII remaining in the blood was measured at respective time points. The mice were administered by subcutaneous injection of DBCOPEG20kDa-scFVIII G4 B3 Az and DBCOPEG30kDa-scFVIII G4 B3 Az at a dose of 180 IU/kg based on FVIII activity; and blood was collected from the retro-orbital venous sinus of each mouse at fixed time points (1 hr, 4 hr, 8 hr, 16 hr, 24 hr, 48 hr, 72 hr). To the collected blood was added an anticoagulant (3.2% sodium citrate) so that the anticoagulant had a final concentration of 10% (v/v). Then, centrifugation was performed to obtain plasma, and the plasma was stored frozen until measurement of FVIII activity. Quantification of the administered substance and quantification of activity of FVIII remaining in the plasma were performed by the CS measurement method as described in Example 1.3. In order to calculate bioavailability obtained upon subcutaneous administration, in Example 4.1, the AUC obtained upon intravenous administration of DBCOPEG20kDa-scFVIII G4 B3 Az and DBCOPEG30kDa-scFVIII G4 B3 Az at a dose of 200 IU/kg was calibrated with 180 IU/kg, which is a dose for subcutaneous administration, so that the bioavailability was calculated. The bioavailability calculated by this calibration is expressed as follows: (AUC_{sc}/AUCᵢᵥ) × (doseᵢᵥ/dose_{sc}) (AUC_{sc}: AUC obtained upon subcutaneous administration; AUCᵢᵥ: AUC obtained upon intravenous administration; doseᵢᵥ: dose for intravenous administration; dose_{sc}: dose for subcutaneous administration). For FVIII and PEG conjugates of FVIII, a reduction rate *in vivo* in the blood depends only on the substance's concentration, which makes it possible to predict the blood concentration obtained upon intravenous administration with calibration of their initial concentration (J Thromb Haemost 2017 15(6): 1106-1114 Population pharmacokinetics of recombinant coagulation factor VIII-SingleChain in patients with severe hemophilia A; ISTH 2017 pharmacokinetics of N8-GP (turoctocog alfa pegol) dosed subcutaneously to non-human primates and prediction of human PK profile). Activity of FVIII remaining in the plasma, which was collected at respective time points from the mouse group subcutaneously administered the sample, is illustrated in FIG. 10. Based on the average values of FVIII activity in the plasma of each mouse group, PK parameters were derived therefrom through the non-compartmental analysis (NCA) method using WinNonlin software, and the results are shown in Table 6. DBCOPEG20kDa-scFVIII G4 B3 Az and DBCOPEG30kDa-scFVIII G4 B3 Az had a terminal half-life of 14.6 hours and 16.9 hours, respectively. The area under the curve (AUC) of Advate® obtained upon subcutaneous administration was less than 1%, whereas DBCOPEG20kDa-scFVIII G4 B3 Az and DBCOPEG30kDa-scFVIII G4 B3 Az had bioavailability of 35% and 43%, respectively, in case of being administered by subcutaneous injection. That is, it was found that the glycoPEGylated scFVIII's according to the present invention had bioavailability remarkably higher than that of the existing FVIII (Advate®), thereby showing that the glycoPEGylated scFVIII's, DBCOPEG20kDa-scFVIII G4 B3 Az and DBCOPEG30kDa-scFVIII G4 B3 Az, could be developed as a subcutaneous formulation, unlike the existing FVIII (Advate®).

Thus, it is inferred that DBCOPEG20kDa-scFVIII G4 B3 Az and DBCOPEG30kDa-scFVIII G4 B3 Az produced by the method of the present invention have high bioavailability in case of being subcutaneously administered for the following reason: in the subcutaneous environment, they are more stable than the existing FVIIII and more completely protected from proteolytic enzymes, and thus have efficient resistance, so that their bioavailability is increased.

**[Table 6]**

| | DBCOPEG20kDa-scFVIII G4 B3 Az | DBCOPEG30kDa-scFVIII G4 B3 Az |
|---|---|---|
| T_{1/2, terminal} (hr) | 14.6±4.4 | 16.9±10.2 |
| Cₘₐₓ (IU/mL) | 1.6±0.5 | 1.6±0.2 |
| AUCₗₐₛₜ (hr^{∗}IU/mL) | 37.9±4.8 | 59.5±14.5 |
| AUC_{INF_ obs} (hr^{∗}IU/mL) | 40.9±3.1 | 65.9±13.0 |
| *Bioavailability (%) | 35 | 43 |

| | | |
|---|---|---|
| *(AUC_{sc}/AUCᵢᵥ) × (doseᵢᵥ/dose_{sc}) | | |

From these results, it was identified that scFVIII modified with polyethylene glycol polymer by the method according to the present invention could remain longer in the body, that is, in blood and subcutaneous tissue, than Advate®, which is the existing FVIII, so that therapeutic efficacy of a drug can be maintained longer.

As described above, the present invention has been described with reference to an embodiment thereof. However, it will be appreciated by those skilled in the art that the present invention may be variously modified and changed by addition, alteration, deletion, or the like of constituent components without departing from the spirit of the present invention described in the claims, and such modifications or changes are also included within the scope of the present invention.

## Claims

1. An *in vivo* long-acting recombinant coagulation factor VIII, comprising a linker which is linked to a sialic acid derivative at the terminus of at least one sugar chain of a coagulation factor VIII, wherein the linker is represented by Formula 1: 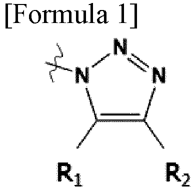 where R₁ and R₂ are each independently C₁₋₈ alkyl, C₆₋₁₂ aryl, C₃₋₆ cycloalkyl, 3- to 10-membered heterocycloalkyl, amino, (amino)C₁₋₆ alkyl, (amino)C₃₋₆ cycloalkyl, (amino)C₆₋₁₂ aryl, (amino)(3- to 10-membered heterocycloalkyl), or 5-to 12-membered heteroaryl (in which the heterocycloalkyl and the heteroaryl may each independently contain at least one heteroatom selected from the group consisting of N, S and O, and may each independently be substituted with at least one substituent selected from the group consisting of halogen, C₁₋₄ alkyl, and CF₃; and the amino may be substituted at least one substituent selected from the group consisting of H, halogen, CN, C₁₋₆ alkyl, haloC₁₋₃ alkyl or C₁₋₆ alkoxycarbonyl), or R₁ and R₂ may be combined with each other to form 8- to 16-membered heterofused ring (in which the heterofused ring may each independently contain at least one heteroatom selected from the group consisting of N, S and O, and may each independently be substituted with at least one substituent selected from the group consisting of halogen, C₁₋₄ alkyl, and CF₃); and
the linker contains a biocompatible polymer connected to at least one of R₁, R₂, or a ring formed when R₁ and R₂ are combined with each other.

2. The *in vivo* long-acting recombinant coagulation factor VIII of claim 1, wherein the biocompatible polymer is at least one selected from the group consisting of polyethylene glycol (PEG), polysialic acid (PSA), polyoxazoline, heparosan, and combinations thereof.

3. The *in vivo* long-acting recombinant coagulation factor VIII of claim 2, wherein the polyethylene glycol has a weight average molecular weight of 5 to 40 kDa.

4. The *in vivo* long-acting recombinant coagulation factor VIII of claim 1, wherein the structure of Formula 1 is at least one selected from the group consisting of Formulas 2 to 18 and isomers thereof: 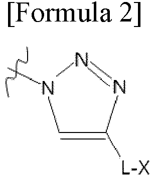 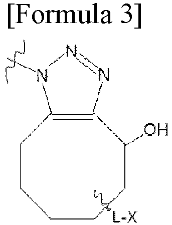 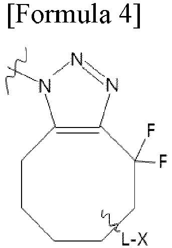 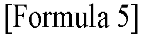 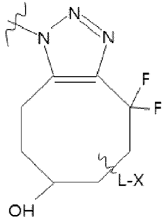 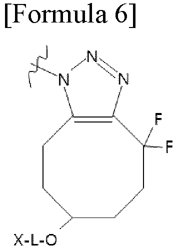 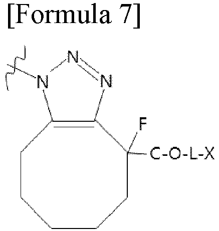 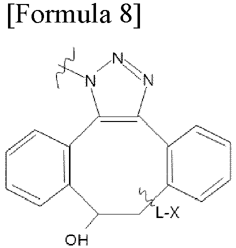 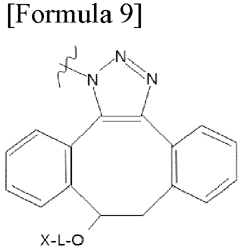 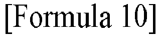 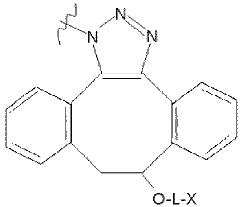 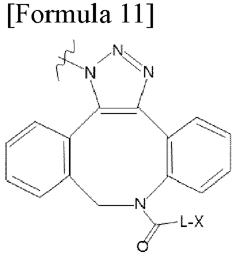 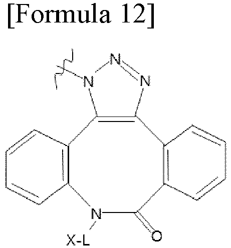 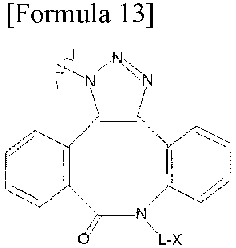 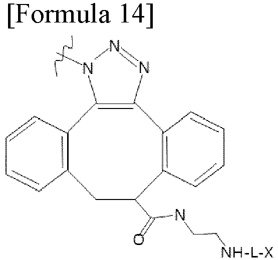 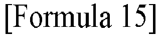 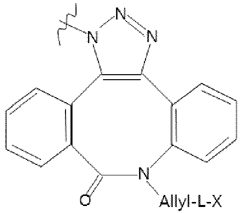 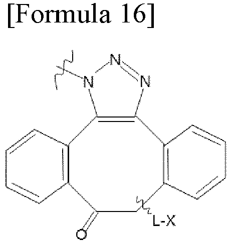 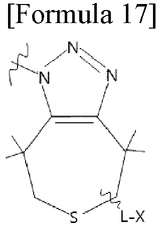 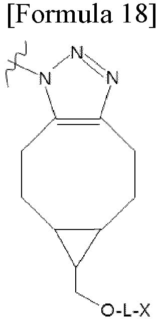 where L may be a single bond, halogen, C₁₋₈ alkyl, C₆₋₁₂ aryl, -CO-,-R₃CONR₃-, -OCONR₃-, or -R₃NCOR₃- (in which R₃ may each independently be selected from H, C₁₋₄ alkyl and C₆₋₁₂ aryl), and X is a biocompatible polymer.

5. The *in vivo* long-acting recombinant coagulation factor VIII of claim 1, wherein the structure of Formula 1 is at least one selected from the group consisting of Formulas 19 to 26: 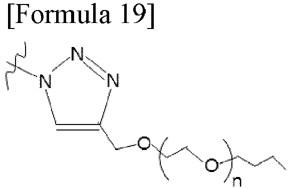 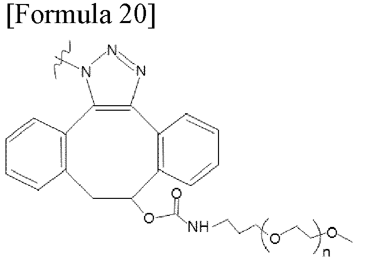 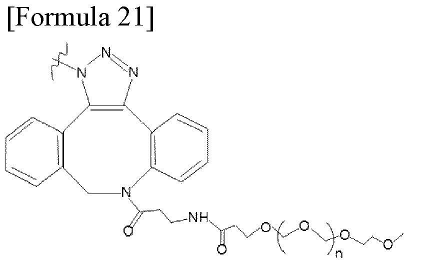 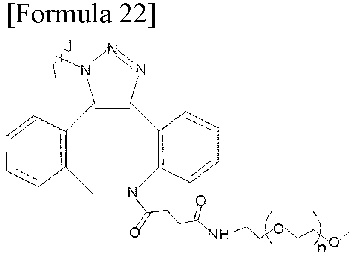 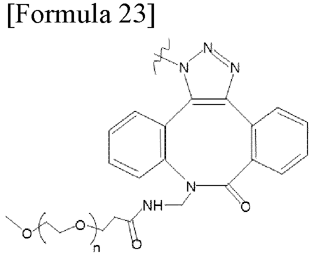 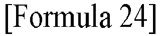 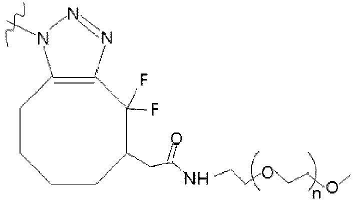 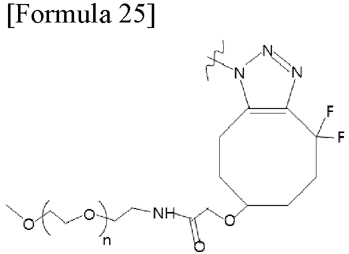 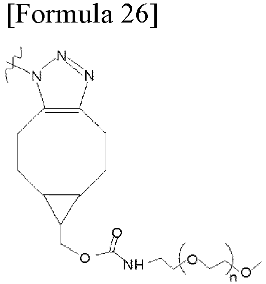 where n is an integer of 100 to 3,000.

6. A method for preparing an *in vivo* long-acting recombinant coagulation factor VIII, comprising the steps of:
(1) introducing, into a host cell, an expression vector that contains nucleotides encoding a coagulation factor VIII; and
(2) culturing the host cell in a culture medium supplemented with a derivative of a sialic acid metabolite having a first functional group attached thereto, to obtain a coagulation factor VIII to which the sialic acid derivative having the first functional group attached thereto is introduced at the terminus of at least one sugar chain.

7. The method of claim 6, wherein the first functional group is an azide group or an alkyne group.

8. The method of claim 6, wherein in step (2), the derivative of the sialic acid metabolite is at least one compound selected from the group consisting of Formulas 27 to 32 and isomers thereof:

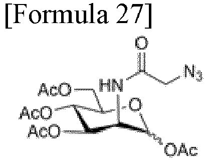

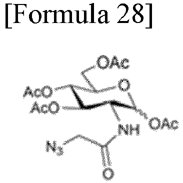

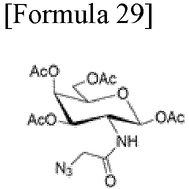

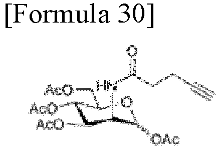

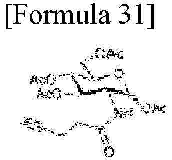

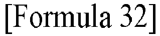

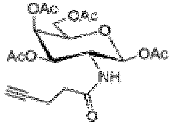

9. The method of claim 6, wherein in step (2), the derivative of the sialic acid metabolite is supplemented in a concentration of 0.1 to 300 uM when the host cell has a concentration of 1.0 × 10⁵ or higher.

10. The method of claim 9, wherein in step (2), the temperature is adjusted to 29°C to 35°C when the derivative of the sialic acid metabolite is supplemented.

11. The method of claim 6, further comprising the steps of:
(3) removing the derivative of the sialic acid metabolite from the culture medium;
(4) adding, to the culture medium, a compound having a second functional group and a biocompatible polymer attached thereto, to allow a click reaction; and
(5) collecting a coagulation factor VIII to which the biocompatible polymer is attached at the terminus of at least one sugar chain.

12. The method of claim 11, wherein the biocompatible polymer is at least one selected from the group consisting of polyethylene glycol (PEG), polysialic acid (PSA), polyoxazoline, heparosan, and combinations thereof.

13. The method of claim 11, wherein the second functional group is an alkyne group or a cycloalkyne group when the first functional group is an azide group; and the second functional group is an azide group when the first functional group is an alkyne group.

14. The method of claim 11, wherein in step (4), the compound having the second functional group and the biocompatible polymer attached thereto is at least one compound selected from the group consisting of Formulas 33 to 49 and isomers thereof: 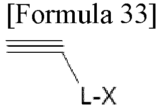 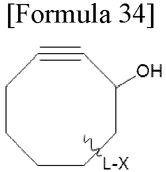 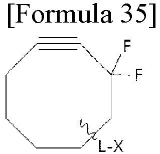 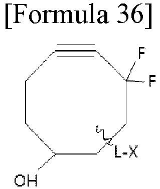 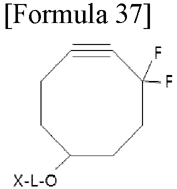 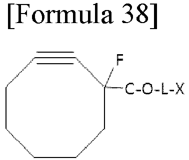 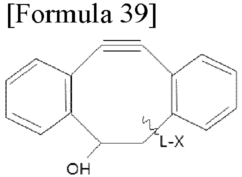 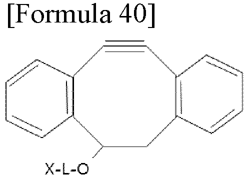 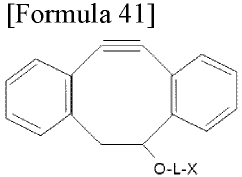 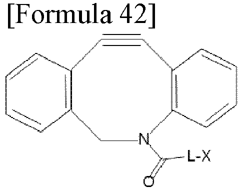 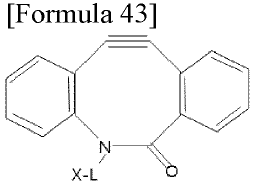 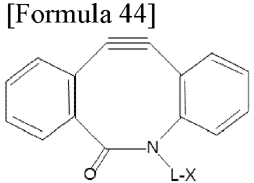 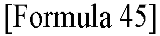 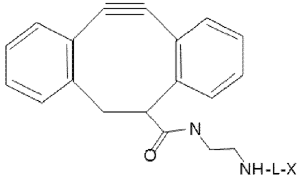 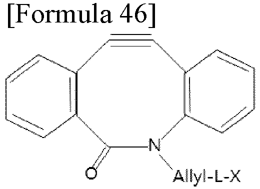 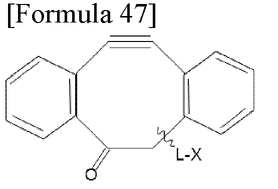 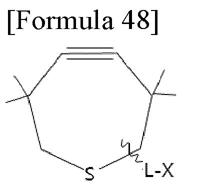 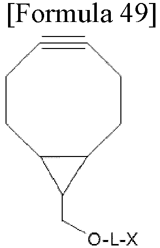 where L may be a single bond, halogen, C₁₋₈ alkyl, C₆₋₁₂ aryl, -CO-,-R₃CONR₃-, -OCONR₃-, or -R₃NCOR₃- (in which R₃ may each independently be selected from H, C₁₋₄ alkyl and C₆₋₁₂ aryl), and X is a biocompatible polymer.

15. The method of claim 6, wherein the *in vivo* long-acting recombinant coagulation factor VIII comprises a linker which is linked to the sialic acid derivative at the terminus of at least one sugar chain of the coagulation factor VIII, wherein the linker is represented by Formula 1: 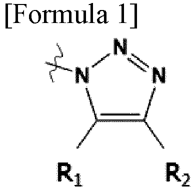 where R₁ and R₂ are each independently C₁₋₈ alkyl, C₆₋₁₂ aryl, C₃₋₆ cycloalkyl, 3- to 10-membered heterocycloalkyl, amino, (amino)C₁₋₆ alkyl, (amino)C₃₋₆ cycloalkyl, (amino)C₆₋₁₂ aryl, (amino)(3- to 10-membered heterocycloalkyl), or 5-to 12-membered heteroaryl (in which the heterocycloalkyl and the heteroaryl may each independently contain at least one heteroatom selected from the group consisting of N, S and O, and may each independently be substituted with at least one substituent selected from the group consisting of halogen, C₁₋₄ alkyl, and CF₃; and the amino may be substituted at least one substituent selected from the group consisting of H, halogen, CN, C₁₋₆ alkyl, haloC₁₋₃ alkyl or C₁₋₆ alkoxycarbonyl), or R₁ and R₂ may be combined with each other to form 8- to 16-membered heterofused ring (in which the heterofused ring may each independently contain at least one heteroatom selected from the group consisting of N, S and O, and may each independently be substituted with at least one substituent selected from the group consisting of halogen, C₁₋₄ alkyl, and CF₃); and
the linker contains a biocompatible polymer connected to at least one of R₁, R₂, or a ring formed when R₁ and R₂ are combined with each other.

16. The method of claim 15, wherein the structure of Formula 1 is at least one selected from the group consisting of Formulas 19 to 26: 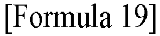 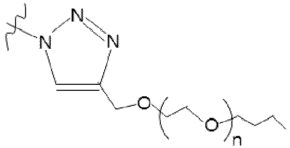 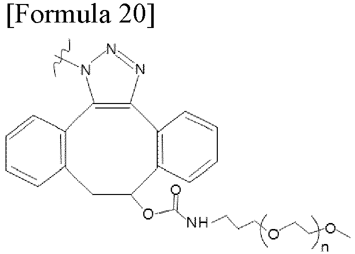 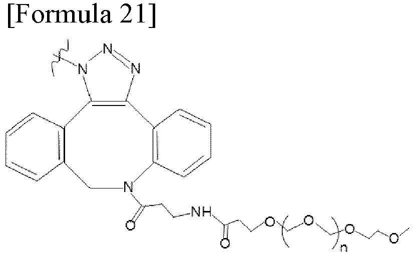 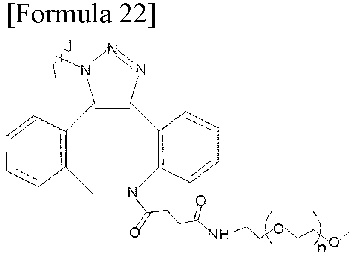 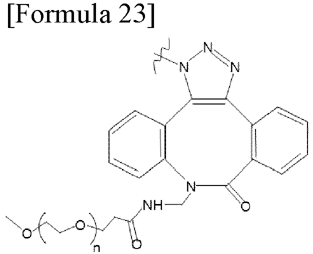 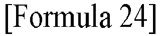 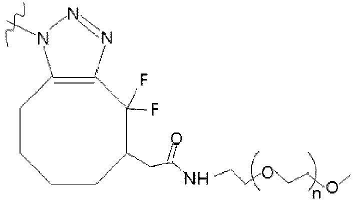 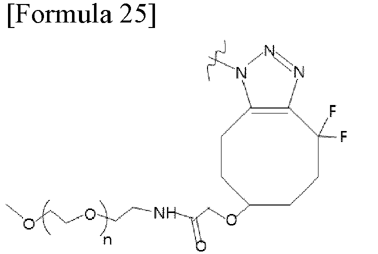 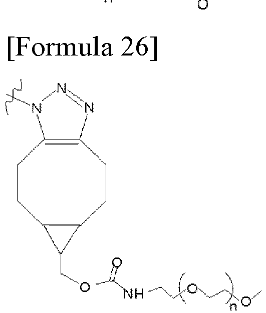 where n is an integer of 100 to 3,000.
